# EUROPEAN PATENT APPLICATION

(11) **EP 4 086 275 A1**
(43) Date of publication of application: **09.11.2022**
(21) Application number: 20910955.2
(22) Date of filing: 09.11.2020
(51) Int. Cl.: C07K 14/47, A61K 9/00

(54) **MULTIMERIZATION DELIVERY SYSTEM FOR INTRACELLULAR DELIVERY OF MOLECULE**

(30) Priority: 31.12.2019 CN 201911414575
(71) Applicant: Xiamen University, Xiamen, Fujian 361005 (CN); Xiamen Innovax Biotech Co., Ltd., Xiamen, Fujian 361022 (CN)
(72) Inventor: GE, Shengxiang, Xiamen, Fujian 361005 (CN); YU, Siyuan, Xiamen, Fujian 361005 (CN); YANG, Han, Xiamen, Fujian 361005 (CN); PAN, Haifeng, Xiamen, Fujian 361005 (CN); REN, Shuling, Xiamen, Fujian 361005 (CN); LI, Tingdong, Xiamen, Fujian 361005 (CN); GUO, Qingshun, Xiamen, Fujian 361005 (CN); XIONG, Junhui, Xiamen, Fujian 361005 (CN); ZHANG, Jun, Xiamen, Fujian 361005 (CN); XIA, Ningshao, Xiamen, Fujian 361005 (CN)
(74) Representative: J A Kemp LLP
(86) International application number: PCT/CN2020/127525
(87) International publication number: WO 2021/135647

(57) **Abstract**

A multimerization delivery system that can be used to deliver a cargo molecule intracellularly. The multimerization delivery system can achieve high-efficiency endocytosis of a cargo molecule and high-efficiency release thereof from an endocytic vesicle, significantly improving the cytoplasmic delivery efficiency of the cargo molecule. Once the cargo molecule is available in the cytoplasm, the cargo molecule can play any role related thereto. The multimerization delivery system provides an effective means for affecting the biological mechanisms and pathways of cells, and can be used in various fields such as research, treatment, and diagnosis.

## Description

### Technical Field

The present invention relates to the field of molecular biology, and more particularly, to a multimerization delivery system for intracellular delivery of a cargo molecule.

### Background Art

Cell membranes as selectively permeable barriers are critical for survival and function of cells. Small molecules can pass through cell membranes either by the natural processes of cells or by direct diffusion of lipid bilayers, but in most cases, the efficient passage of intracellular cargoes such as active biological macromolecules through cytoplasmic membranes remains a major obstacle to cellular transport process. Therefore, a molecular transport tool that can effectively improve the transport efficiency of an intracellular cargo to a living cell is extremely important for its application in the fields of biomedicine and so on.

At present, the use of cell-penetrating peptides (CPPs) to mediate the entry of biological macromolecules into cells is one of the research hotspots in the field of biomedicine. CPPs are generally polypeptides of 5-30 amino acids, which can mediate biological macromolecules including proteins to penetrate cell membranes and enter cells by chemical cross-linking, fusion expression or non-covalent binding. Cell-penetrating peptides have been used in various fields of biomedicine due to the advantages of low dosage, short transport time, controllable dosage, simple operation, low immune response and low side effects when mediating the entry of biological macromolecules into cells.

Although CPPs have the above advantages in transporting biological macromolecules, there are still some limitations, mainly including their own low delivery efficiency and further reduced delivery efficiency in the presence of serum.

Previous studies have shown that only 1% of biomacromolecules carried by CPPs can escape from endocytic vesicles successfully, and most of them are eventually directed to lysosomes for degradation. Therefore, the endosomal escape efficiency of intracellular cargo is also a key limiting factor for the intracellular delivery process of CPPs, which determines the overall delivery efficiency. A large number of studies have been devoted to solving the problem of low delivery efficiency of CPPs. For improving the endosomal escape efficiency, the main strategy is to destroy the integrity of vesicle membrane during the process of maturation and acidification, so that its contents (including delivered intracellular cargo) can be released into the cytoplasm. Currently, the most effective way is to use pH-sensitive fusogenic peptides derived from viruses, bacteria, animals and plants or humans. The pH-sensitive peptides contain a certain proportion of hydrophobic amino acids and undergo dramatic conformational changes at low pH. During the process of maturation and acidification of endocytic vesicles after CPPs-mediated endocytosis, once the pH value drops to a critical point, the pH-sensitive peptides coupled to CPPs undergo a conformational change and bind to the lipid bilayers of vesicle membrane. As a result, the integrity of phospholipid bilayer membrane is violently disturbed, small pores are formed on it, or the vesicle membrane is ruptured, and finally the transported biological macromolecules are released into the cytoplasm. However, subsequent studies found that although the endocytic vesicle escape efficiency of the intracellular delivery system of macromolecules using CPPs fused with pH-sensitive peptides was indeed improved, a considerable part of the transported macromolecules still remained in the endocytic vesicles (obvious spotted distribution could be observed when fluorescent protein was used as the transported macromolecule).

Another factor limiting the application of cell-penetrating peptides is serum intolerance, that is, under the condition of high serum concentration, CPPs cannot sufficiently interact with the cell membrane due to the electrostatic interaction between the positive charge of CPPs and the negative charge on hemoglobin, resulting in a sharp drop in their delivery efficiency, which also severely limits the wide application of CPPs in vivo.

To sum up, although a lot of work has been done to improve the efficiency of CPPs-mediated macromolecular entry into cells, the effect is still not satisfactory. The efficiency of entry into cells remains a major limiting factor for the widespread application of CPPs in the field of development of biomacromolecular drugs that target intracellular targets. At the same time, serum tolerance is also a problem that must be solved for using CPPs in drug development, while there is no research report concerning this problem.

### Contents of the present invention

After extensive experiments and repeated explorations, the inventors of the present application unexpectedly find that the introduction of a multimerization domain can significantly improve the endocytosis efficiency of cargo molecules and significantly improve serum tolerance. In addition, the combination of pH-sensitive peptides and specific protease recognition sequences can significantly improve the release of cargo molecules from endocytic vesicles, further improve the cytoplasmic delivery efficiency of cargo molecules, and enable the cargo molecules to fully exert their corresponding biological functions. Based on these findings, the present inventors developed a multimerization delivery system to achieve efficient cytoplasmic delivery.

### Fusion polypeptide

Accordingly, in a first aspect, the present invention provides a fusion polypeptide comprising a multimerization domain sequence, a cell-penetrating peptide, a pH-sensitive fusogenic peptide, and a protease recognition sequence.

In the present invention, the term "multimerization domain" refers to any polypeptide or protein capable of multimerizing (i.e., forming a biomolecular complex) several copies of the fusion polypeptide described herein. In certain embodiments, the multimerization domain is a homomultimerization domain that aggregate identical fusion polypeptides to form a multimer.

In certain embodiments, the multimerization domain can be a dimerization domain, trimerization domain, tetramerization domain, or substantially any higher-order multimerization domain, so long as the domain is capable of facilitating an interaction between at least two domains (and polypeptides in which the domains constitute parts).

In certain embodiments, the multimerization domain is selected from the group consisting of: leucine zipper, NOE (SEQ ID NO: 3), GCN4-P1 (SEQ ID NO: 4), Delta (SEQ ID NO: 5) and any combination thereof.

In certain embodiments, the multimerization domain is selected from leucine zipper.

In certain embodiments, the multimerization domain sequence comprises a sequence set forth in SEQ ID NO: 1 or 2.

In the present invention, the term "pH-sensitive fusogenic peptide" is used interchangeably with "pH-sensitive peptide", which refers to a class of polypeptides that is capable of undergoing a conformational change under acidic conditions (e.g., pH<6.5), thereby promoting its fusion with an endocytic vesicle membrane. During the process of maturation and acidification of endocytic vesicle after a pH-sensitive peptide is endocytosed by a cell, once the pH value drops to a critical point, such peptide undergoes a conformational change and bind to the lipid bilayer of the vesicle membrane, thereby violently disturbing the integrity of the phospholipid bilayer membrane. As a result, the integrity of phospholipid bilayer membrane is violently disturbed, small pores are formed on it, or the vesicle membrane is ruptured, and finally the transported biological macromolecules are released into the cytoplasm. Such polypeptides are well known in the art and are described, for example, in Varkouhi, Amir K., et al. Journal of Controlled Release 151.3 (2011): 220-228; Erazo-Oliveras A, Muthukrishnan N, Baker R, et al. Pharmaceuticals, 2012, 5(11): 1177-1209, which are incorporated herein by reference in their entirety.

The pH-sensitive peptide that can be used in the fusion protein of the present invention may be selected from, or derived from, the following proteins or polypeptides:
viral protein sources: HA2 (influenza virus) and its mutants KALA, GALA; penton base (adenovirus or rhinovirus), gp41 (HIV), L2 (papillomavirus), envelope protein (West Nile virus);
bacterial protein sources: Listeriolysin O (LLO), Pneumococcal pneumolysin (PLO), Streptococcal streptolysin O (SLO), Diphtheria toxin, *Pseudomonas aeruginosa* exotoxin A, Shiga toxin, cholera toxin;
plant protein sources: Ricin, Saporin, Gelonin toxin;
human/animal protein sources: human calcitonin, fibroblast growth factor receptor, melittin;
synthetic peptides: (R-Ahx-R) (4) AhxB, penetratin (pAntp), EB1, bovine prion protein (bPrPp), sweet arrow peptide (SAP), poly(L-histidine), proline-rich peptide.

In certain embodiments, the pH-sensitive fusogenic peptide is selected from influenza virus HA2 (SEQ ID NO: 36) or a mutant thereof, melittin (SEQ ID NO: 39), and any combination thereof. In certain embodiments, the mutant of influenza virus HA2 is selected from INF7 (SEQ ID NO: 12), KALA (SEQ ID NO: 37) or GALA (SEQ ID NO: 38).

In certain embodiments, the pH-sensitive fusogenic peptide comprises INF7. In certain embodiments, the pH-sensitive fusogenic peptide comprises or consists of the following sequence: SEQ ID NO: 12.

In the present invention, the term "cell-penetrating peptide (CPP)" is also referred to as "cell penetrating peptide", "protein translocation domain (PTD)", "Trojan horse peptide" or "transduction peptide" and the like, which refer to a polypeptide capable of promoting cellular uptake of various molecules (e.g., various macromolecules including proteins or nucleic acids). Such polypeptides are well known in the art and are described, for example, in Stewart KM, et al. Org Biomol Chem. 2008 Jul 7;6(13):2242-55, and Chinese patent application CN101490081A (all of which are incorporated herein by reference in their entirety); or can be obtained by methods known in the art, such as the method described in US Patent Application US2008/0234183, which is incorporated herein by reference in its entirety.

CPPs that can be used in the fusion protein of the present invention include, but are not limited to: cationic type: Penetratin, HIV-TAT-47-57, HIV-1 Rev 34-50, FHV coat-35-49, Oligoarginines (R9-R12), CCMV Gag-7-25, S413-PV, VP22, BP16, DPV3, DPV6, FAH coat, Protamine 1, human cJun, Engrailed-2, Islet-1, HoxA-13, TP10, etc; amphipathic type: transportan, Transportan 10, Pep-1, MPGα, MPGβ, CADY, Pepfect6, Pepfect14, Pepfect15, NickFect, Hel, sC18, pVEC, ARF(1-22), *YTA2,* PARI (*Palmitoyl-SFLLRN*)*,* F2Pal10 (*Palmitoyl-SFLLRN*)*,* BPrPp(1-30), hLF peptide (19-40), Buforin 2, Crotamine, *Azurin p18*, hCT peptide (18-32), S413-PVrev et al; hydrophobic type: Kaposi's sarcoma fibroblast growth factor, signal peptide of Ig light chain derived from *Caiiman crocodylus,* integrin β3 fragment, Grb2-SH2 domain, HIV-1 gp41 (1-23), HBV translocation motif, sperm-egg fusion protein (89-111), human calcitonin (9-32), Pep-7, C105Y, K-FGF, etc.

In addition, the CPP used in the fusion protein of the present invention can also be selected from polypeptide sequences having a sequence identity of about 60, 70, 80, 90, 95, 99% or 100% to any of the polypeptide sequences described above, as long as the polypeptide sequences still retain biological activity thereof, i.e., promoting cellular uptake of molecules.

In certain embodiments, the cell-penetrating peptide is selected from the group consisting of Penetratin (SEQ ID NO: 40), Tat derived peptide, Rev(34-50) (SEQ ID NO: 42), VP22 (SEQ ID NO: 43), transportan (SEQ ID NO: 44), Pep-1 (SEQ ID NO: 45), Pep-7 (SEQ ID NO: 46), and any combination thereof. In certain embodiments, the Tat-derived peptide is selected from Tat(48-60) (SEQ ID NO: 14) or Tat(47-57) (SEQ ID NO: 41).

In certain embodiments, the cell-penetrating peptide comprises a Tat-derived peptide, such as Tat(48-60). In certain embodiments, the cell-penetrating peptide comprises or consists of the following sequence: SEQ ID NO:14.

In certain embodiments, the protease is selected from furin and/or lysosomal cysteine protease. In certain embodiments, the protease recognition sequence is selected from the group consisting of a furin recognition sequence, a lysosomal cysteine protease recognition sequence, and combinations thereof.

In certain embodiments, the furin recognition sequence comprises or consists of the following sequence: R-X₁-X₂-R^{↓} (SEQ ID NO: 47), wherein X₁ is any amino acid, X₂ is K or R, and ↓ indicates cleavage site.

In certain embodiments, the furin recognition sequence comprises or consists of the following sequence: R-R-X₁-X₂-R^{↓} (SEQ ID NO: 48).

In certain embodiments, X₁ is selected from alanine (A), arginine (R), aspartic acid (D), cysteine (C), glutamine (Q), glutamic acid (E), histidine (H), isoleucine (I), glycine (G), aspartic acid (N), leucine (L), lysine (K), methionine (M), phenylalanine (F), proline (P), serine (S), threonine (T)), tryptophan (W), tyrosine (Y) and valine (V).

In certain embodiments, the furin recognition sequence comprises or consists of the following sequence: RRHKR^{↓} (SEQ ID NO: 49).

In certain embodiments, the furin recognition sequence comprises or consists of the following sequence: QSVASSRRHKR^{↓}FAGV (SEQ ID NO: 8).

In certain embodiments, the lysosomal cysteine protease is selected from the group consisting of cathepsin B, cathepsin C, cathepsin X, cathepsin S, cathepsin L, cathepsin D, or cathepsin H.

In certain embodiments, the lysosomal cysteine protease is cathepsin L.

In certain embodiments, the cathepsin L recognition sequence comprises or consists of the following sequence: NNTHDLVGDVRLAGV (SEQ ID NO: 10).

In certain embodiments, the protease recognition sequence comprises a furin recognition sequence and a cathepsin L recognition sequence. In certain embodiments, the protease recognition sequence is a single-chain polypeptide comprising a furin recognition sequence and a cathepsin L recognition sequence from the N-terminus to the C-terminus, or comprising a cathepsin L recognition sequence and a furin recognition sequence from the N-terminus to the C-terminus.

In certain embodiments, the protease recognition sequence comprises RRHKR (SEQ ID NO: 49) and NNTHDLVGDVRLAGV (SEQ ID NO: 10). In certain embodiments, the protease recognition sequence comprises SEQ ID NO:8 and SEQ ID NO:10.

In some embodiments, the fusion polypeptide comprises, from the N-terminus to the C-terminus: the cell-penetrating peptide, the pH-sensitive fusogenic peptide and the protease recognition sequence, and the multimerization domain sequence is located at the N-terminus or C-terminus of the fusion polypeptide, or between any two adjacent domains described above. In certain exemplary embodiments, the multimerization domain sequence is at the N-terminus of the fusion polypeptide, or at the C-terminus of the fusion polypeptide.

In other embodiments, the fusion polypeptide comprises, from the N-terminus to the C-terminus: the pH-sensitive fusogenic peptide, the cell-penetrating peptide, the protease recognition sequence, and the multimerization domain sequence is located at the N-terminus or C-terminus of the fusion polypeptide, or between any two adjacent domains described above. In certain exemplary embodiments, the multimerization domain sequence is at the N-terminus of the fusion polypeptide, or at the C-terminus of the fusion polypeptide.

In certain embodiments, the protease recognition sequence comprises the furin recognition sequence and the cathepsin L recognition sequence from the N-terminus to the C-terminus. In certain embodiments, the protease recognition sequence comprises the cathepsin L recognition sequence and the furin recognition sequence from the N-terminus to the C-terminus.

In certain embodiments, any adjacent domains contained in the fusion polypeptide are optionally linked by a peptide linker. In certain embodiments, the peptide linker is (GₘS)ₙ, wherein m is selected from an integer of 1 to 4 (e.g., 1, 2, 3, or 4) and n is selected from an integer of 1 to 3 (e.g., 1, 2 or 3). In certain embodiments, the peptide linker is SEQ ID NO:50. In certain embodiments, the peptide linkers between any adjacent domains may be the same or different.

In certain exemplary embodiments, the fusion polypeptide comprises the sequence set forth in any one of SEQ ID NOs: 16 to 18.

In a second aspect, the present invention provides a multimer of the fusion polypeptide of the first aspect.

In certain embodiments, the multimer is a dimer, trimer or tetramer. In certain embodiments, the multimer is a dimer.

In certain embodiments, the multimer is a homomultimer.

### Fusion protein

In a third aspect, the present invention provides a fusion protein, which comprises the fusion polypeptide of the first aspect, and an additional polypeptide.

In certain embodiments, the additional polypeptide comprises a detectable label, such as an enzyme, fluorescent protein, or biotin, and the like.

In certain embodiments, the additional polypeptide comprises an epitope tag, a protein sequence encoded by reporter gene, and/or a nuclear localization signal (NLS) sequence.

Epitope tags that can be used in the present invention are well known to those skilled in the art, examples of which include but are not limited to His, V5, FLAG, HA, Myc, VSV-G, Trx, etc., and those skilled in the art know how to select an appropriate epitope tag for the desired purpose (e.g., purification, detection, or tracking). In certain exemplary embodiments, the additional polypeptide comprises a His-tag.

Reporter gene sequences that can be used in the present invention are well known to those skilled in the art, examples of which include, but are not limited to, GST, HRP, CAT, GFP, HcRed, DsRed, CFP, YFP, BFP, and the like.

Nuclear localization signal (NLS) sequences that can be used in the present invention are well known to those skilled in the art, examples of which include, but are not limited to, NLS of SV40 virus large T antigen. In certain exemplary embodiments, the NLS sequence is set forth in SEQ ID NO:22.

In certain exemplary embodiments, the additional polypeptide is a zinc finger protein (e.g., ZFP9), or a protein phosphatase (e.g., Ppmlb). In certain exemplary embodiments, the zinc finger protein comprises an NLS sequence.

In certain embodiments, the additional polypeptide is a nucleic acid binding domain sequence.

In certain embodiments, the nucleic acid binding domain sequence is a zinc finger protein (e.g., ZFP9).

In certain embodiments, the nucleic acid binding domain sequence comprises the sequence set forth in SEQ ID NO:31.

In certain exemplary embodiments, when the additional polypeptide is a zinc finger protein (e.g., ZFP9), the multimerization domain sequence comprises the sequence set forth in SEQ ID NO:2.

In certain exemplary embodiments, the fusion protein comprises the sequence set forth in any one of SEQ ID NOs: 19 to 21.

In certain embodiments, the fusion protein comprises, from the N-terminus to the C-terminus: the cell-penetrating peptide, pH-sensitive fusogenic peptide, protease recognition sequence, and an additional polypeptide, and the multimerization domain sequence is located at the N-terminus or C-terminus of the fusion protein, or between any two adjacent domains described above. In certain exemplary embodiments, the multimerization domain sequence is located at the N-terminus of the fusion protein, or at the C-terminus to the protease recognition sequence. Preferably, the protease recognition sequence comprises the furin recognition sequence and the cathepsin L recognition sequence from the N-terminus to the C-terminus, or comprises the cathepsin L recognition sequence and the furin recognition sequence from the N-terminus to the C-terminus.

In certain embodiments, the fusion protein comprises, from the N-terminus to the C-terminus: the pH-sensitive fusogenic peptide, cell-penetrating peptide, protease recognition sequence, and an additional polypeptide; and, the multimerization domain sequence is located at the N-terminal or C-terminal of the fusion protein, or between any two adjacent domains described above. In certain exemplary embodiments, the multimerization domain sequence is located at the N-terminus of the fusion protein, or at the C-terminus of the protease recognition sequence. Preferably, the protease recognition sequence comprises the furin recognition sequence and the cathepsin L recognition sequence from the N-terminus to the C-terminus, or comprises the cathepsin L recognition sequence and the furin recognition sequence from the N-terminus to the C-terminus.

In certain embodiments, the additional polypeptide is fused to the C-terminus of the fusion polypeptide.

In certain embodiments, the additional polypeptide has a molecular weight of less than 10000 Da, for example, less than 5000 Da, less than 3000 Da, or less than 1000 Da.

In a fourth aspect, the present invention provides a multimer of the fusion protein of the third aspect.

In certain embodiments, the multimer is a dimer, trimer or tetramer. In certain embodiments, the multimer is a dimer.

In certain embodiments, the multimer is a homomultimer.

### Preparation of multimer

The fusion polypeptide or fusion protein of the present invention can be prepared by various methods known in the art, for example, by genetic engineering methods (recombinant techniques), or by chemical synthesis methods (e.g., Fmoc solid-phase method). The fusion protein of the present invention is not limited by the manner in which it is produced.

Accordingly, in another aspect, the present invention provides an isolated nucleic acid molecule comprising a nucleotide sequence encoding the fusion polypeptide or fusion protein of the present invention.

In another aspect, the present invention provides a vector (e.g., a cloning vector or an expression vector) comprising the isolated nucleic acid molecule as described above. In certain embodiments, the vector is, for example, a plasmid, cosmid, phage, and the like.

In another aspect, the present invention provides a host cell comprising the isolated nucleic acid molecule or the vector as described above. Such host cell includes, but is not limited to, prokaryotic cell such as E. coli cell, and eukaryotic cell such as yeast cell, insect cell, plant cell, and animal cell (e.g., mammalian cell, such as mouse cell, human cell, etc.).

In another aspect, there is provided a method for preparing the fusion polypeptide or fusion protein of the present invention, which comprises, culturing the host cell as described above under conditions that allow expression of the protein, and recovering the fusion polypeptide or fusion protein from a culture of the cultured host cell, wherein the fusion polypeptide or fusion protein exists in the form of a multimer.

In another aspect, there is provided a method for preparing the multimer of the present invention, which comprises, culturing the host cell as described above under conditions that allow expression of the protein, and recovering the multimer from a culture of the cultured host cell.

### Complex

In a fifth aspect, the present invention provides a complex, which comprises the multimer of the second aspect and a cargo molecule; or, which comprises the multimer of the fourth aspect and a cargo molecule. The cargo molecule can be any molecule.

In certain embodiments, the cargo molecule is selected from the group consisting of protein, nucleic acid, carbohydrate, lipid, chemical compound, and any mixture thereof.

In certain embodiments, the cargo molecule has a molecular weight of less than 10000 Da, for example, less than 5000 Da, less than 3000 Da, or less than 1000 Da.

In certain embodiments, the cargo molecule comprises a detectable label, such as an enzyme, radionuclide, fluorescent dye, chemiluminescent substance, or biotin, and the like.

In certain embodiments, the cargo molecule comprises a pharmaceutically active agent.

### Peptide or protein

In some embodiments, the cargo molecule is a peptide or protein. In certain embodiments, the cargo molecule is fused to the fusion polypeptide or fusion protein that forms the multimer. In certain embodiments, the complex comprises the multimer of the second aspect.

In certain embodiments, the complex comprises a single-chain polypeptide, wherein:
(i) the single-chain polypeptide comprises from the N-terminus to the C-terminus: the cell-penetrating peptide, the pH-sensitive fusogenic peptide, the protease recognition sequence and the cargo molecule; and, the multimerization domain sequence is located at the N-terminus or C-terminus of the fusion protein, or between any two adjacent domains described above; preferably, the protease recognition sequence comprises the furin recognition sequence and the cathepsin L recognition sequence from the N-terminus to the C-terminus, or comprises the cathepsin L recognition sequence and the furin recognition sequence from the N-terminus to the C-terminus; or,
(ii) the fusion protein comprises from the N-terminus to the C-terminus: the pH-sensitive fusogenic peptide, the cell-penetrating peptide, the protease recognition sequence and the cargo molecule; and, the multimerization domain sequence is located at the N-terminus or C-terminus of the fusion protein, or between any two adjacent domains described above; preferably, the protease recognition sequence comprises the furin recognition sequence and the cathepsin L recognition sequence from the N-terminus to the C-terminus, or comprises the cathepsin L recognition sequence and the furin recognition sequence from N-terminus to C-terminus; or,
(iii) the cargo molecule is fused to the C-terminus of the fusion polypeptide.

### Nucleic acid

In other embodiments, the cargo molecule is a nucleic acid. In certain embodiments, the nucleic acid is selected from the group consisting of DNA molecule, RNA molecule, siRNA, antisense oligonucleotide, ribozyme, aptamer, and any combination thereof.

In certain embodiments, the complex comprises the multimer of the fourth aspect, wherein the fusion protein constituting the multimer comprises a nucleic acid binding domain sequence. In certain embodiments, the nucleic acid binding domain sequence is a zinc finger protein (e.g., ZFP9). In certain embodiments, the nucleic acid binding domain sequence comprises the sequence set forth in SEQ ID NO:31. In certain exemplary embodiments, when the additional polypeptide is a zinc finger protein (e.g., ZFP9), the multimerization domain sequence comprises the sequence set forth in SEQ ID NO:2.

### Linking manner

In certain embodiments, the cargo molecule is fused, chemically coupled or non-covalently linked to the multimer (or the fusion polypeptide or fusion protein constituting the multimer).

In some embodiments, the multimer of the second or fourth aspect (or the fusion polypeptide or fusion protein constituting the multimer) is fused to a cargo molecule. In certain embodiments, the cargo molecule is a peptide or protein.

In some embodiments, the multimer of the second or fourth aspect (or the fusion polypeptide or fusion protein constituting the multimer) is chemically coupled to the cargo molecule. The "chemical coupling" refers to a bonding obtained in a chemical reaction between a reactive group contained in the multimer (or the fusion polypeptide or fusion protein constituting the multimer) and a reactive group contained in the cargo molecule, and the two moieties are joined by a covalent bond after the chemical reaction. Before the above chemical reaction (coupling reaction), the multimer, the cargo molecule, or both can be modified in a separate reaction with a linker molecule so that they each contain the reactive group required for the chemical coupling. The choice of linker molecule used to modify the multimer or cargo molecule depends on the coupling strategy used.

In certain embodiments, the covalent bond is a disulfide bond, phosphodiester bond, phosphorothioate bond, amide bond, amine bond, thioether bond, ether bond, ester bond, or carbon-carbon bond.

In certain embodiments, the cargo molecule is coupled to the C-terminus of the multimer.

In certain embodiments, the cargo molecule is a nucleic acid.

In other embodiments, the multimer of the second or fourth aspect is non-covalently linked to the cargo molecule.

In certain embodiments, the multimer is electrostatically conjugated to the cargo molecule.

In certain embodiments, the cargo molecule is a nucleic acid.

### Preparation of complex

In some embodiments, when the complex comprises the multimer and cargo molecule which are chemically-coupled, the complex of the present invention may be obtained by the following exemplary method: mixing the multimer with the cargo molecule under conditions allowing the chemical reaction between the reactive groups separately contained in the multimer and the cargo molecule, so that the two moieties are covalently linked. In certain embodiments, the method further comprises: using a linker molecule to modify the multimer, the cargo molecule, or both so that they respectively contain the reactive groups required for the chemical reaction described above. In certain embodiments, the cargo molecule is a nucleic acid.

In other embodiments, when the complex comprises the multimer and the cargo molecule conjugated by electrostatic interaction, the complex of the present invention can be obtained by the following exemplary method: (1) mixing the multimer of the present invention with the cargo molecule to form a mixture; and (2) incubating the mixture so that the multimer and the cargo molecule form a complex. In certain embodiments, the cargo molecule is a nucleic acid. In such embodiments, the fusion protein constituting the multimer preferably further comprises a nucleic acid binding domain sequence.

### Use and method

The multimer or complex of the present invention is capable of significantly improving the endocytosis efficiency of cargo molecules, enabling efficient release of cargo molecules from endocytic vesicles, and once the cargo molecules are available in the cytoplasm, they can play any role associated with them. Thus, the multimer or complex of the present invention can be used as an intracellular delivery agent for further researches as well as therapeutic and diagnostic applications.

Accordingly, in another aspect, the present invention provides a pharmaceutical composition, which comprises the fusion polypeptide, fusion protein, multimer, complex, isolated nucleic acid molecule, vector or host cell of the present invention, and a pharmaceutically acceptable carrier and/or excipient.

In certain embodiments, the pharmaceutical composition comprises the complex of the fifth aspect.

In certain embodiments, the cargo molecule contained in the complex is a pharmaceutically active agent or a detectable label. Such label can be used for diagnosis, for studying drug disposition (e.g., absorption, distribution, metabolism, excretion), for studying efficacy or side effects of a treatment or drug, and the like.

In another aspect, the present invention also relates to a use of the fusion polypeptide, fusion protein, multimer, complex of the present invention, or the isolated nucleic acid molecule, vector or host cell comprising a nucleotide sequence encoding the fusion polypeptide or fusion protein in the manufacture of a medicament for treating a disease; wherein the cargo molecule contained in the complex can treat the disease. In another aspect, the present invention also relates to a method for treating a disease, comprising administering to a subject in need thereof the fusion polypeptide, fusion protein, multimer, complex of the present invention, or the isolated nucleic acid molecule, vector or host cell comprising a nucleotide sequence encoding the fusion polypeptide or fusion protein; wherein the cargo molecule contained in the complex is capable of treating the disease.

In certain embodiments, the disease is a disease associated with programmed necrosis, wherein the cargo molecule comprises protein phosphatase 1B (Ppm1b). In certain embodiments, the disease associated with programmed necrosis comprises liver injury (e.g., drug-induced liver injury, such as acute liver injury caused by acetaminophen APAP), inflammatory disease (e.g., systemic inflammatory response syndrome SIRS), ischemia-reperfusion injury and/or neurodegenerative disease. In certain embodiments, the disease is a disease associated with programmed necrosis caused by TNF-α, for example, SIRS, a systemic inflammatory response syndrome caused by TNF-α.

The fusion polypeptide, fusion protein, multimer, complex or pharmaceutical composition of the present invention can be in any form known in the medical field, for example, can be in the form of tablet, pill, suspension, emulsion, solution, gel, Capsule, powder, granule, elixir, lozenge, suppository, injection (including injection solution, lyophilized powder), inhalant, spray, etc. The preferred dosage form depends on the intended mode of administration and therapeutic use.

The fusion polypeptide, fusion protein, multimer, complex or pharmaceutical composition of the present invention can be administered by any suitable method known in the art, including but not limited to oral, rectal, parenteral or topical administration.

An exemplary route of administration is oral administration. The liquid dosage form for oral administration includes pharmaceutically acceptable emulsion, microemulsion, solution, suspension, syrup, elixir, and the like. In addition to the active ingredient, the liquid dosage form may contain an inert diluent commonly used in the art, such as water or other solvent, solubilizer and emulsifier, such as ethanol, isopropanol, ethyl acetate, ethyl acetate, benzyl alcohol, benzyl benzoate ester, propylene glycol, 1,3-butanediol, dimethylformamide, oil (such as cottonseed oil, peanut oil, corn oil, germ oil, olive oil, castor oil, and sesame oil), glycerin, tetrahydrofurfuryl alcohol, polyethylene glycol, fatty acid sorbitan ester, and mixture thereof. In addition to the inert diluent, the liquid dosage form for oral administration may also contain an adjuvant such as wetting agent, emulsifying agent and suspending agent, sweetening agent, flavoring agent and perfuming agent, and the like. The solid dosage form for oral administration includes capsule, tablet, pill, lozenge, powder, granule, and the like. In addition to the active ingredient, the solid dosage form may contain a pharmaceutically acceptable inert excipient or carrier, such as filler (e.g., lactose, sucrose, glucose, mannitol, starch, microcrystalline cellulose, galactose, crospovidone, and calcium sulfate); binder (e.g., carboxymethyl cellulose, alginate, gelatin, polyvinylpyrrolidone, sucrose, and acacia); humectant (e.g., cetyl alcohol, and glyceryl monostearate); disintegrant (e.g., agar, calcium carbonate, starch, alginic acid, sodium carboxymethyl cellulose, sodium carboxymethyl starch); lubricant (e.g., talc, calcium stearate, magnesium stearate, solid polyethylene glycol, lauryl sodium sulfate); and mixture thereof.

The fusion polypeptide, fusion protein, multimer, complex or pharmaceutical composition of the present invention can also be administered by a non-oral route.

Thus, another exemplary route of administration is parenteral administration, for example, subcutaneous injection, intravenous injection, intraperitoneal injection, intramuscular injection, intrasternal injection, and infusion. The dosage form for parenteral administration may be a preparation for injection, including solution for injection, sterile powder for injection, or concentrated solution for injection. In addition to the active ingredient, the dosage form for injection may contain a pharmaceutically acceptable carrier such as sterile water, Ringer's solution and isotonic sodium chloride solution, and an appropriate additive such as antioxidant, buffer and bacteriostatic agent.

Another exemplary route of administration is topical administration, for example, transdermal administration (e.g., administration via a transdermal patch or iontophoresis device), intraocular administration, or intranasal or inhalation administration. The dosage form for transdermal administration may be a topical gel, spray, ointment and cream. In addition to the active ingredient, the topical dosage form may contain an ingredient that enhances absorption or penetration of the active ingredient through the skin or other action area.

Another exemplary route of administration is rectal administration. The dosage form for rectal administration may be a suppository.

In addition, other carrier materials and modes of administration known in the pharmaceutical art can also be used. The fusion polypeptide, fusion protein, multimer, complex or pharmaceutical composition of the present invention can be prepared by any known pharmaceutical techniques, such as effective method of formulation and administration.

In another aspect, the present invention provides a kit, which comprises the fusion polypeptide, fusion protein, multimer, complex, isolated nucleic acid molecule, vector or host cell of the present invention. In certain embodiments, the kit further comprises an instruction for transfection and/or intracellular delivery. In certain embodiments, the kit is used for transfection and/or intracellular delivery of a cargo molecule (e.g., nucleic acid, peptide or protein, carbohydrate, lipid, chemical compound, and any mixture thereof). In certain embodiments, the cell is a mammalian cell, such as human cell.

In another aspect, the present invention also relates to a use of the fusion polypeptide, fusion protein, multimer, complex, isolated nucleic acid molecule, vector or host cell of the present invention as a delivery reagent (e.g., transfection reagent or intracellular delivery reagent). In certain embodiments, the delivery reagent is used for intracellular delivery of a cargo molecule (e.g., nucleic acid, peptide or protein, carbohydrate, lipid, chemical compound, and any mixture thereof). In certain embodiments, the cell is a mammalian cell, such as human cell.

In another aspect, the present invention provides a method for delivering a cargo molecule into a cell, comprising: contacting the cell with the complex of the fifth aspect, wherein the cargo molecule is a cargo molecule contained in the complex.

In certain embodiments, the contacting of the cell with the complex is performed in vivo.

In certain embodiments, the contacting of the cell with the complex is performed ex vivo.

In certain embodiments, the contacting of the cell with the complex is performed in vitro.

In certain embodiments, the cell is a eukaryotic cell, such as mammalian cell, for example, human cell.

### Definition of terms

In the present invention, unless otherwise specified, the scientific and technical terms used herein have the meanings commonly understood by those skilled in the art. In addition, the operation steps of cell culture, biochemistry, nucleic acid chemistry, immunology laboratory, etc. used herein are all routine steps widely used in the corresponding fields. Meanwhile, for a better understanding of the present invention, definitions and explanations of related terms are provided below.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell by transformation, transduction or transfection, so that a genetic material element carried by it can be expressed in the host cell. The vector is well known to those skilled in the art and includes, but is not limited to: plasmid; phagemid; cosmid; artificial chromosome, such as yeast artificial chromosome (YAC), bacterial artificial chromosome (BAC) or PI derived artificial chromosome (PAC); phage such as λ phage or M13 phage and animal virus. The animal virus that can be used as the vector includes, but is not limited to, retrovirus (including lentivirus), adenovirus, adeno-associated virus, herpesvirus (e.g., herpes simplex virus), poxvirus, baculovirus, papillomavirus, papovavirus (e.g.,SV40). A vector may contain a variety of elements that control expression, including, but not limited to, promoter sequence, transcription initiation sequence, enhancer sequence, selection element, and reporter gene. Additionally, the vector may also contain an origin of replication.

As used herein, the term "host cell" refers to a cell that can be used to introduce a vector, including, but not limited to, prokaryotic cell such as *E. coli* or *Bacillus subtilis,* fungal cell such as yeast cell or *Aspergillus,* etc., insect cell such as *S2 Drosophila* cell or Sf9, or animal cell such as fibroblast, CHO cell, COS cell, NSO cell, HeLa cell, BHK cell, HEK 293 cell or human cell.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The twenty conventional amino acids involved herein are expressed in routine manners. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which are well-known in the art (see, for example, Remington's Pharmaceutical Sciences. Edited by Gennaro AR, 19th ed. Pennsylvania: Mack Publishing Company, 1995), and include, but are not limited to: pH adjuster, surfactant, ionic strength enhancer, agent for maintaining osmotic pressure, agent for delaying absorption, diluent, adjuvant, preservative, stabilizer, etc. For example, pH adjusting agent includes but is not limited to phosphate buffer. Surfactant includes but is not limited to cationic, anionic or non-ionic surfactant, such as Tween-80. Ionic strength enhancer includes but is not limited to sodium chloride. Agent for maintaining osmotic pressure includes but is not limited to, sugar, NaCl and the like. Agent for delaying absorption includes but is not limited to monostearate and gelatin. Diluent includes but is not limited to water, aqueous buffer (e.g., buffered saline), alcohol and polyol (e.g., glycerol), etc. Adjuvant includes but is not limited to aluminum adjuvant (e.g., aluminum hydroxide), Freund's adjuvant (e.g, complete Freund's adjuvant), and the like. Preservative includes but is not limited to various antibacterial and antifungal agents, such as thimerosal, 2-phenoxyethanol, paraben, trichloro-tert-butanol, phenol, sorbic acid and the like. Stabilizer has the meaning commonly understood by those skilled in the art, which can stabilize a desired activity of an active ingredient in a drug (for example, the inhibitory activity of PSD-95 ubiquitination), including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dried whey, albumin or casein) or degradation product thereof (e.g., lactalbumin hydrolysate), etc.

As used herein, the term "treatment" refers to a method performed in order to obtain a beneficial or desired clinical result. For the purpose of the present invention, the beneficial or desired clinical result includes, but is not limited to, alleviating symptom, narrowing the scope of disease, stabilizing (i.e. not aggravating) the state of disease, delaying or slowing the progress of disease, and alleviating symptoms (either partially or completely), no matter detectable or not detectable. In addition, "treatment" can also refer to prolonging survival time as compared to an expected survival time (if not receiving treatment).

As used herein, the term "subject" refers to a mammal, such as a primate, such as a human.

### Beneficial effects of the present invention

The multimerization delivery system of the present invention can significantly improve the endocytosis efficiency and endocytic vesicle release efficiency of a cargo molecule, thereby significantly improving the cytoplasmic delivery efficiency of the cargo molecule, so that the cargo molecule can fully exert its corresponding biological function. In particular, the multimerization delivery system of the present invention can significantly improve serum tolerance, thereby enabling in vivo delivery. Therefore, the delivery system of the present invention provides an effective means for influencing biological mechanisms and pathways of cells, can be used in many fields such as research, treatment, diagnosis, etc., and thus has broad application prospects and clinical value.

The embodiments of the present invention will be described in detail below with reference to the drawings and examples, but those skilled in the art will understand that the following drawings and examples are only used to illustrate the present invention, rather than limit the scope of the present invention. Various objects and advantageous aspects of the present invention will become apparent to those skilled in the art from the accompanying drawings and the following detailed description of the preferred embodiments.

### Brief Description of the Drawings

FIG. 1 shows the clone design for the multimerization delivery system-eGFP based on leucine zipper.
FIG. 2 shows the effect of leucine zipper on the state of protein aggregate.
FIG. 3 shows the evaluation of the endocytosis efficiency and vesicle escape efficiency of the delivery system after the addition of leucine zipper domain.
FIG. 4 shows the evaluation of the endocytosis efficiency of the delivery system under serum conditions after the addition of leucine zipper domain.
FIG. 5 shows the clone design for the delivery system-eGFP based on multiple multimerization domains.
FIG. 6 shows the fluorescence microscopy imaging of the effects of various multimerization domains on endocytosis efficiency.
FIG. 7 shows the evaluation of the effects of various multimerization domains on endocytic efficiency.
FIG. 8 shows the clone design for TINNeL-eGFP/GFPβ1-10.
FIG. 9 shows the evaluation of the endocytosis efficiency of TINNeL delivery system.
FIG. 10 shows the evaluation of the vesicle escape efficiency of TINNeL delivery system.
FIG. 11 shows the evaluation of the delivery efficiency of TINNeL delivery system under different serum concentrations.
FIG. 12 shows a schematic diagram of the transport mechanism of the multimerization delivery system of the present invention.
FIG. 13 shows the clone constructions for the protein of TINNeL-Ppm1b delivery system and other control proteins.
FIG. 14 shows the effect of TINNeL-Ppmlb on inhibiting TNF-α-induced cell necrosis.
FIG. 15 shows the survival rate of mice after inhibition of TNF-α-induced death by TINNeL-Ppm1b.
FIG. 16 shows the HE staining of mouse SIRS cecum caused by TNF-α after inhibition by TINNeL-Ppm1b.
FIG. 17 shows the injury scores of mouse SIRS cecum caused by TNF-α after inhibition by TINNeL-Ppm1b.
FIG. 18 shows the changes in ALT/AST levels after TINNeL-Ppmlb treatment of APAP-induced acute liver injury in mice.
FIG. 19 shows the survival rate of mice with TINNeL-Ppmlb treatment against death caused by APAP.
FIG. 20 shows the clone constructions for the protein of TINNeL-ZFP9 delivery system and other control proteins.
FIG. 21 shows a schematic diagram of the eukaryotic expression plasmid structure for ZFP9 delivery.
FIG. 22 shows the flow cytometry analysis about the transduction efficiency of red fluorescent protein expression plasmid by TINNeL-ZFP9.
FIG. 23 shows the flow cytometry analysis about the red fluorescent protein expression plasmid transduced by TINNeL-ZFP9 and X-tremeGNEN under serum conditions.
FIG. 24 shows the image of mice about fluorescence expression of Luciferase plasmid transduced by TINNeL-ZFP9.
FIG. 25 shows fluorescence microscopy observations of transduction of GFPβ1-10 by the delivery system.

### Sequence information

Information of the partial sequences involved in the present invention is provided in Table 1 below.

**Table 1: Description of sequences**

| SEQ ID NO | Description | SEQ ID NO | Description |
|---|---|---|---|
| 1 | Leucine zipper-1 | 26 | Nucleic acid sequence encoding GFPβ1-10-NLS |
| 2 | Leucine zipper-2 | 27 | Nucleic acid sequence encoding Histone-H3 |
| 3 | NOE multimerization domain | 28 | Nucleic acid sequence encoding GFPβ11 |
| 4 | GCN4-P1 multimerization domain | 29 | Ppm1b |
| 5 | Delta multimerization domain | 30 | Nucleic acid sequence encoding Ppm1b |
| 6 | Nucleic acid sequence encoding leucine zipper-1 | 31 | ZFP9-NLS |
| 7 | Nucleic acid sequence encoding leucine zipper-2 | 32 | Nucleic acid sequence encoding ZFP9-NLS |
| 8 | Furin recognition sequence Ne | 33 | tdTomato coding sequence |
| 9 | Nucleic acid sequence encoding Ne | 34 | ZFP9 binding site 6^{∗} |
| 10 | CTSL recognition sequence N | 35 | Luciferase coding gene |
| 11 | Nucleic acid sequence encoding N | 36 | Influenza virus HA2 |
| 12 | INF7 | 37 | KALA |
| 13 | Nucleic acid sequence encoding INF7 | 38 | GALA |
| 14 | Tat(48-60) | 39 | Melittin |
| 15 | Nucleic acid sequence encoding Tat(48-60) | 40 | Penetratin |
| 16 | Fusion protein TINL | 41 | HIV-TAT(47-57) |
| 17 | Fusion protein TINeL | 42 | HIV-1 Rev(34-50) |
| 18 | Fusion protein TINNeL | 43 | VP22 |
| 19 | Fusion protein TINL-ZFP9 | 44 | Transportan |
| 20 | Fusion protein TINeL-ZFP9 | 45 | Pep-1 |
| 21 | Fusion protein TINNeL-ZFP9 | 46 | Pep-7 |
| 22 | NLS | 47 | Furin recognition sequence-1 |
| 23 | eGFP | 48 | Furin recognition sequence-2 |
| 24 | Nucleic acid sequence encoding eGFP | 49 | Furin recognition sequence-3 |
| 25 | GFPβ1-10-NLS | 50 | Peptide linker |

### Examples

The present invention will now be described with reference to the following examples, which are intended to illustrate, but not limit, the present invention.

Unless otherwise specified, the molecular biology experimental methods and immunoassay methods in the present invention were performed basically by referring to the methods described in J. Sambrook et al., Molecular Cloning: A Laboratory Manual, 2nd Edition, Cold Spring Harbor Laboratory Press, 1989, and F. M. Ausubel et al., Refined Molecular Biology Laboratory Manual, 3rd Edition, John Wiley & Sons, Inc., 1995; the restriction enzymes were used according to the conditions recommended by the product manufacturer. Those skilled in the art appreciate that the examples describe the present invention by way of example and are not intended to limit the scope of the present invention as claimed.

The sources of the main reagents involved in the following examples are as follows:
The materials required for clone construction related reagents were as follows: DNA polymerase (TaKaRa, R040A), DNA recovery kit (TianGen, DP214-03), plasmid mini kit (TianGen, DP103-03), plasmid Maxi kit (QIAGEN), 12663), Gibson assembly Master Mix (NEB, E2611L), DNA marker (ThmeroFisher, SM0331), agarose (Biowest, BW-R0100);
The materials required for large-scale protein expression were as follows: peptone (BiSIGMA-ALDRICH, T7293-1KG), yeast powder (OXOID, LP0021B), sodium chloride (Xilong Chemical Industry, 10011012AR), IPTG (Inalco, 1758-1400);
The media required for protein purification were as follows: SP SEPHAROSE FAST FLOW (GE Healthcare, 17-0729-01), NI SEPHAROSE (GE Healthcare, 17-5268-02);
The reagents required for protein purification and preservation were as follows: glycerol/glycerol/C₃H₈O₃ (SIGMA-ALDRICH, G5516), KCl (Xilong Chemical Industry, 1002007), Na₂HPO₄ · 12H₂O (Xilong Chemical Industry, 1001067AR), KH₂PO₄ (Xilong Chemical Industry, 1002048AR500), imidazole (SIGMA-ALDRICH, V900153), Tris base (Seebio, 183995), glucose (Xilong Chemical Industry, 1064008AR500), BCA protein concentration assay kit (Thermo Scientific, 23227);
Reagents required for cell culture: FBS (GIBCO, 10099-133), DMEM (GIBCO, 11965092), trypsin (AMRESCO, 0458);
Reagents required for lentiviral packaging and infection: lentiviral packaging plasmids: pCMV-VSV-G (Addgene, 8454), pRSV-Rev (Addgene, 12253), pMDLg/pRRE (Addgene, 12251); X-tremeGENE transfection reagent (Roche, 06366244001), Puromycin (InvivoGen, ant-pr-5), Blasticidin (InvivoGen, ant-bl-5b), polybrene (Santa Cruz, sc-134220);
The GFPβ1-10, ZFP9, Ppmlb, dsRed, mCherry, Histone-H3 related plasmids used in the experiment were synthesized by Sangon Bio, and the plasmid used for amplifying the Cas9 sequence was pCasKP-hph (Addgene, 117232);

### Other reagents: TNF-α (Novoprotein, CF09), PI (ThmeroFisher, P3566)

Cell lines: HEK-293T (human kidney epithelial cells), L929 (mouse fibroblasts) were purchased from ATCC.

Example 1: Establishment and performance evaluation of TL multimerization delivery system

In this example, a multimerization delivery system was established by using leucine zipper. The effect of the multimerization on endocytosis efficiency was observed by delivering green fluorescent protein eGFP. The effect of the multimerization on vesicle escape efficiency was observed by delivering GFP β1-10-NLS (briefly referred as GFP β-10) based on the Split-GFP evaluation method. Thereby, comprehensive evaluation of the effects of the multimerization on the delivery efficiency of the delivery system was carried out. At the same time, by adding serum (FBS) to the system, the difference in endocytosis efficiency before and after multimerization was observed to evaluate the change of serum tolerance.

### 1.1 Construction of expression vector for multimerization delivery system (TAT-Leu Zipper)-cargo molecule complex

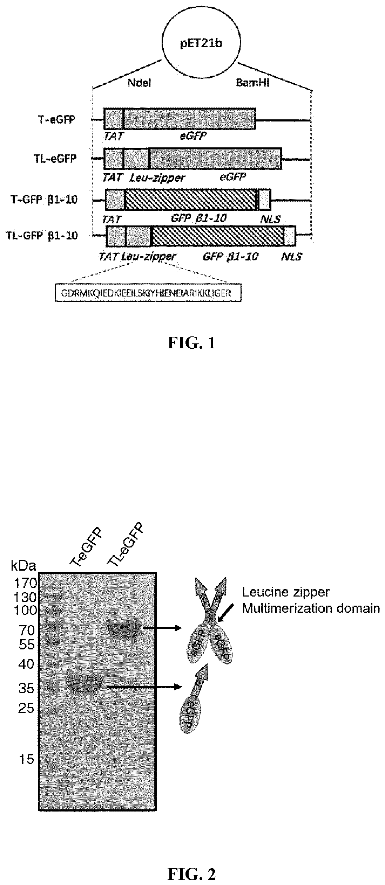

An expression vector of recombinant protein containing TAT, Leu-Zipper (leucine zipper), and cargo molecule was constructed, and the structures of the nucleic acids encoding each recombinant protein were shown in FIG. 1. The amino acid sequence of each component from the N-terminus to the C-terminus was shown in the table below. The construction method was as follows: first, the nucleic acid sequences encoding TAT, leucine zipper, and cargo molecule (eGFP or GFP β1-10) in the delivery system were obtained by PCR amplification, and all these parts were linked by multiple rounds of PCR, and during the last round of PCR, the *NdeI* restriction site and the overlap on upstream of the corresponding *NdeI* restriction site in pET21b(+) were introduced at the 5' end of the fragment through the forward primer, and the *BamHI* restriction site and the overlap on downstream of the corresponding *BamHI* restriction site in pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly.

**Table 2: Components contained in the delivery system-cargo molecule complex**

| Complex name | N-terminal → C-terminal components and their sequences | | |
|---|---|---|---|
| | CPP | Leucine zipper | Cargo |
| T-eGFP | Tat SEQ ID NO:14 | None | eGFP SEQ ID NO: 23 |
| TL-eGFP | | SEQ ID NO: 1 | |
| T-GFP β1-10 | | None | GFP β1-10-NLS SEQ ID NO: 25 |
| TL-GFP β1-10 | | SEQ ID NO: 1 | |

1.2 Expression and purification of multimerization delivery system-cargo molecule complex:
The expression plasmid described in 1.1 was transformed into the expression strain *E.coli* BL21 (DE3); a single colony was picked from the transformed plate and inoculated into 5 ml of LB liquid medium containing ampicillin resistance and cultivated overnight, and then 1 ml of bacterial liquid overnight cultured was taken, and transferred to 500 ml of LB liquid medium containing ampicillin resistance, and cultivated at 37°C and 180 rpm until the bacterial liquid had OD₆₀₀ of about 0.6, and then the inducer IPTG was added to a final concentration of 0.2 mM, and induced at 25°C for 8 hours; after the end of the induced expression, the cells were collected after centrifugation under 7000g at 4°C for 10 minutes; then the cells were resuspended with 10 ml of protein purification equilibration buffer (PBS, 5% glycerol), and disrupted by ultrasonication. Then, the supernatant was taken by centrifugation and loaded onto the SP strong cation chromatography column of the protein purification system; then the protein purification system was used to elute the target protein with protein elution buffer (PBS+0.2M NaCl~PBS+0.6M NaCl to remove impurity proteins, PBS+1.6M NaCl to collect the elution product). The protein concentration can be determined according to spectrophotometer or BCA protein concentration assay kit. Each purified fusion protein was aliquoted and stored at -20°C.

### 1.3 Evaluation of multimerization effect of leucine zipper on delivery system-cargo complex

TAT-eGFP (T-eGFP) obtained in 1.2 above and leucine zipper-bearing TAT-Leu-eGFP (TL-eGFP) were subjected to non-reducing polyacrylamide gel electrophoresis to analyze its aggregate formation state. The results were shown in FIG. 2. The size of T-eGFP protein in monomer state was about 35kD, while the protein size of TL-eGFP after the addition of leucine zipper was at 70kD. This experiment showed that the leucine zipper could promote the delivery system protein to form dimer.

### 1.4 Evaluation of endocytosis efficiency of multimerization delivery system-eGFP complex

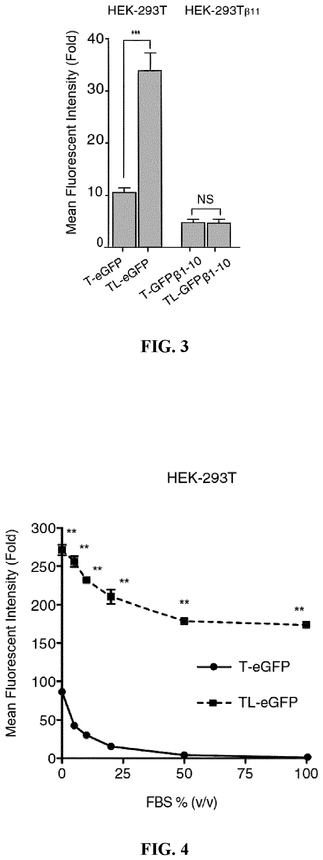

HEK-293T cells were inoculated into a 12-well cell culture plate and cultured overnight. Before protein treatment, it was ensured that the number of cells in each well was about 2^{∗}10⁶; the cells were rinsed three times with serum-free DMEM medium, and then TL-eGFP and T-eGFP at 5 µM respectively were added to serum-free medium; after 3 h incubation, rinsing was performed three times with pre-cooled serum-free DMEM containing 20 U/mL heparin to remove the proteins that were adsorbed on the cell membrane and failed to enter the cells, and the cells were collected to perform flow cytometry analysis. The results were shown in FIG. 3. The intracellular fluorescence intensity of the dimerized protein TL-eGFP was 4-5 times that of the monomer (T-eGFP), which proved that the multimerization process improved the endocytosis efficiency of the delivery system.

### 1.5 Evaluation of vesicle escape efficiency of multimerization delivery system-GFP β1-10 complex

In the Split-GFP system, the 11 β-sheets of GFP were split into a large fragment (β1-10) and a small fragment (β11), both of which lost their fluorescent activity but could spontaneously associate and restore GFP fluorescence property if they met. Based on this, HEK293T cells that could stably express Histone-β11 was constructed, and the GFPβ1-10 with nuclear entry signal (NLS) was used as Cargo for the fusion expression with the multimerization delivery system to be evaluated, and then subjected to transduction of the stable cell line. When GFPβ1-10 was transduced by the delivery system, it could bind to GFPβ11 and generate complete GFP only after successfully escaping from the endocytic vesicle and entering the cytoplasm or nucleus, so that the endocytic vesicle escape efficiency could be evaluated by the proportion and relative fluorescence intensity of ratio of GFP.

### 1.5.1 Construction of HEK293T-GFPβ11 cell line

(1) Construction of lentiviral plasmid of GFPβ11 cell line:
   The coding sequence of Histone-H3 (SEQ ID NO: 27) was amplified by PCR, and the coding sequence of GFPβ11 (SEQ ID NO: 28) was short and directly designed in the upstream primer. The components were ligated through multiple rounds of PCR, and in the last round of PCR, the *Hind III*restriction site and the overlap on upstream of the corresponding *Hind III*restriction site on Lenti vector were introduced at the 5' end of the fragment through the forward primer, and the *BamHI* restriction site and the overlap on downstream of the corresponding *BamHI* restriction site on Lenti vector were introduced at the 3' end of the fragment through the reverse primer. The Lenti plasmid was subjected to double digestion with *Hind III* and *BamH I.* The insert fragments with overlaps were ligated to the digested Lenti vector by GIBSON assembly.
(2) Lentiviral packaging, infection and resistance screening of cell lines:
   HEK-293T cells were inoculated into a 6-well plate and cultured overnight. Before plasmid transfection, it was ensured that the number of cells per well was about 2^{∗}10⁷/ml; before transfection, the cells were transferred into serum-free DMEM medium; 1.5 µg of Lenti recombinant plasmid, 0.75 µg of pMDL plasmid, 0.45 µg of pVSV-G plasmid, 0.3 µg of pREV (mass ratio of 5:3:2:1) were added to 300 µl of serum-free DMEM, followed by slowly blowing. 9 µl (1:3) of X-tremeGENE transfection reagent was added and slowly blown, allowed to stand at room temperature for 15 min, and added dropwise into the cell supernatant; after 8 h, culturing was continued by changing the medium to DMEM containing 10% FBS; after 60 h, the cell culture supernatant was collected for later infection.
   HEK-293T cells were inoculated into a 12-well plate and cultured overnight. Before lentivirus infection, it was ensured that the number of cells in each well was about 2^{∗}10⁶/ml (50% density); the original cell culture supernatant was discarded, 300 µl of lentivirus (moi=3) and 700 µl of 10% FBS DMEM were added, polybrene was added at a concentration of 10 µg/ml, and the cell plate was centrifuged at 2500 rpm for 30 min under aseptic conditions, and then continued to culture.
   After lentivirus infection for 48 hours, the cells were passaged at a ratio of 1/3, and puromycin was added at a concentration of 2.5 µg/ml for resistance screening; the positive cells obtained by screening were cloned to obtain HEK-293T-Hitone-GFPβ11 monoclonal cell line.

### 1.5.2 Detection of vesicle escape efficiency by Split-GFP system

The above HEK-293T Histone H3-β11 cells (HEK-293T β11 for short) were inoculated into a 12-well cell culture plate and cultured overnight. Before protein treatment, it was ensured that the number of cells in each well was about 2^{∗}10⁶; serum-free DMEM medium was used to rinse the cells three times, and then TL-GFP β1-10 and T-GFP β1-10 at 5 µM respectively were added to the serum-free medium; after incubation for 3 h, a pre-cooled serum-free DMEM containing 20 U/ml heparin was used to perform rinsing three times to remove the proteins that were adsorbed on the cell membrane and failed to enter the cells, and then the culturing was continued for another 9 h after changing the medium to DMEM medium containing serum. And the cells were collected to perform flow cytometry analysis. The results showed that the vesicle escape efficiency of the dimerized protein TL-GFP β1-10 did not change in comparison with the monomeric T-GFP β1-10 (FIG. 3).

### 1.6 Evaluation of tolerance of multimerization delivery system to serum conditions

HEK-293T cells were inoculated into a 12-well cell culture plate and cultured overnight. Before protein treatment, it was ensured that the number of cells in each well was about 2^{∗}10⁶; the cells were rinsed three times with serum-free DMEM medium, then TL-eGFP and T-eGFP at 5 µM respectively were added to serum-free, 5%, 10%, 20%, 50%, 100% FBS DMEM media; after incubation for 3 h, pre-cooled serum-free DMEM containing 20 U/mL heparin was used for rinsing to remove the proteins that were adsorbed on the cell membrane and failed to enter the cells, then the medium was replaced with serum-free DMEM medium, and the cells were collected for flow cytometry analysis. The results were shown in FIG. 4. With the increase of serum content, the transduction efficiencies of TL-eGFP and T-eGFP decreased, but the transduction efficiency of TL-eGFP was relatively less affected by serum, and at the condition of 50% FBS, there was almost no detectable intracellular green fluorescence signal after the cells ere transduced by T-eGFP, while the dimerized protein TL-eGFP still had a transduction efficiency of 70% compared to that without serum. It could be seen that the multimerization not only improved the endocytosis efficiency of the protein, but also conferred a higher serum tolerance to the protein.

### 1.7 Effect of different multimerization domains on endocytosis efficiency of delivery system

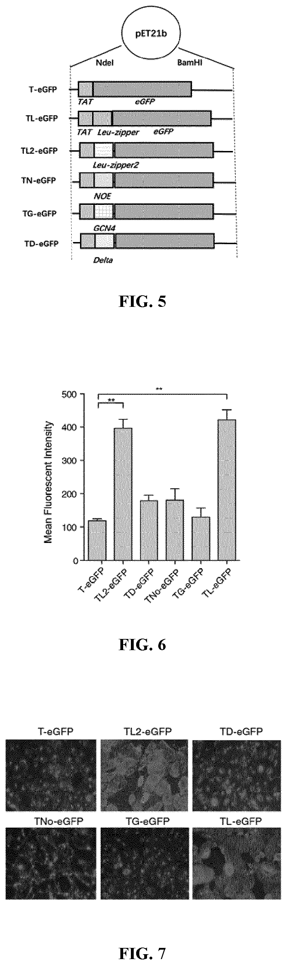

In this example, the effects of different multimerization domain sequences on the endocytosis efficiency of the delivery system were compared. The components of the delivery system-cargo molecule complexes comprising different multimerization domains were shown in the table below. The expression vector for expressing the above-mentioned complexes was prepared by the methods described in the above 1.1 to 1.2, and the above-mentioned complexes was expressed and purified. The schematic diagram of the constructed plasmid was shown in FIG. 5.

**Table 3: Delivery systems comprising different multimerization domains**

| Name of complex | N-terminal → C-terminal components and sequences thereof | | |
|---|---|---|---|
| | CPP | Multimerization domain | Cargo |
| T-eGFP | Tat SEQ ID NO:14 | None | eGFP SEQ ID NO: 23 |
| TL-eGFP | | Leucine zipper SEQ ID NO: 1 | |
| TL2-eGFP | | Leucine zipper 2 SEQ ID NO: 2 | |
| TN-eGFP | | NOE SEQ ID NO: 3 | |
| TG-eGFP | | GCN4-P1 SEQ ID NO: 4 | |
| TD-eGFP | | Delta SEQ ID NO: 5 | |

MA104 cells were inoculated into a 12-well cell culture plate and cultured overnight. Before protein treatment, it was ensured that the number of cells in each well was about 2^{∗}10⁶; the cells were rinsed three times with serum-free DMEM medium, and then T-eGFP, TL-eGFP, TL2-eGFP, TN-eGFP, TG-eGFP and TD-eGFP at 5 µM respectively were added to serum-free medium; after incubation for 3 h, the cells were rinsed three times with pre-cooled serum-free DMEM containing 20 U/mL heparin to remove the proteins that were adsorbed on the cell membrane but failed to enter the cells, after the medium was replaced with serum-free medium, fluorescence imaging was performed, and the cells were collected for flow cytometry analysis. The fluorescence imaging results were shown in FIG. 6. The intracellular green fluorescence intensity of the T-eGFP group was very low, while the intracellular fluorescence intensity of the other experimental groups added with the multimerization domain was significantly increased. The results of flow cytometry analysis were shown in FIG. 7. Different multimerization domains all could enhance the average fluorescence intensity of eGFP mediated by TAT, that was, improved the efficiency of TAT-mediated endocytosis, and the leucine zipper-based TL-eGFP and TL2-eGFP had the most obvious effect.

### Example 2: Establishment and performance evaluation of TINNeL multimerization delivery system

In this example, based on the multimerization delivery system of Example 1, pH-sensitive peptide (INF7) and endocytic vesicle protease-specific cleavage site (CTSL protease: N, Furin protease: Ne) were further added to construct TINNEL delivery system, and its endocytic efficiency and vesicle escape efficiency were evaluated.

### 2.1 Construction of expression vector for TINNEL-cargo molecule complex

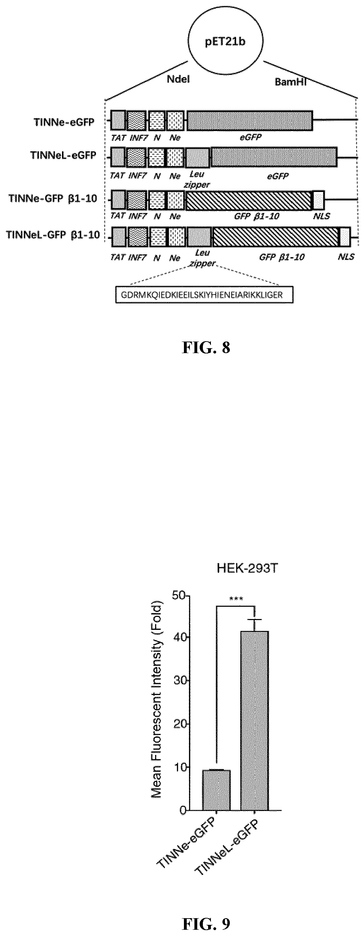

The expression vector of TINNEL-cargo molecule recombinant protein was constructed, the amino acid sequence of each component contained in each recombinant protein from the N-terminus to the C-terminus was shown in the following table. The construction method was as follows: first, the nucleic acid sequences encoding TAT, INF7, leucine zipper, N, Ne, and cargo molecule (eGFP or GFP β1-10) in the delivery system were obtained by PCR amplification, and these parts were ligated through multiple rounds of PCR, and in the last round of PCR, the *NdeI* restriction site and the overlap on upstream of the corresponding *NdeI* restriction site on pET-21b(+) were introduced at the 5' end of the fragment through the forward primer, and the *BamHI* restriciton site and the overlap on downstream of the corresponding *BamHI* restriction site on pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly. The schematic diagram of the plasmid was shown in FIG. 8.

**Table 4: Components of delivery system-cargo molecule complex**

| | N-terminal → C-terminal components and sequences thereof | | | | |
|---|---|---|---|---|---|
| Name of complex | CPP | pH-sensitive peptide | Protease recognition sequence | Multimerization domain | Cargo molecule |
| TINNe-eGFP | TAT SEQ ID NO: 14 | INF7 SEQ ID NO: 12 | N+Ne | None | eGFP SEQ ID NO: 23 |
| TINNeL-eGFP | | | | Leucine zipper SEQ ID NO: 1 | |
| TINNe-GFP β1 - 10 | | | | None | GFP β1-10-NLS SEQ ID NO: 25 |
| TINNeL-GFP β1-10 | | | | Leucine zipper SEQ ID NO: 1 | |

### 2.2 Expression and purification of TINNeL-cargo complex

First, the expression plasmid obtained in 2.1 was transformed into the expression strain *E.coli* BL21 (DE3); a single colony was picked from the transformed plate and inoculated into 5ml of LB liquid medium containing ampicillin resistance for overnight culture, and then 1ml of the bacterial solution overnight cultured was transferred to 500 mL of LB liquid medium containing ampicillin resistance, and cultivated at 37°C and 180 rpm until the bacterial liquid had an OD₆₀₀ of about 0.6, and then the inducer IPTG was added to reach a final concentration of 0.2 mM, and the induction was performed at 25 °C for 8 hours; after the end of induced expression, the cells were collected by centrifugation under 7000g at 4°C for 10 minutes; then the cells were resuspended with 10 ml of protein purification equilibration buffer (PBS, 5% glycerol), and disrupted by ultrasonication. Then, the supernatant was collected by centrifugation and loaded onto the SP strong cation chromatography column of the protein purification system; then the protein purification system was used to elute the target protein with protein elution buffer (PBS+0.2M NACl~PBS+0.7M NaCl to remove impurity proteins, PBS+1.8M NaCl to collect the elution product). The protein concentration could be determined according to spectrophotometer or BCA protein concentration assay kit. Each purified fusion protein was aliquoted and stored at - 20°C.

### 2.3 Evaluation of intracellular delivery efficiency of TINNeL delivery system

Evaluation of endocytosis efficiency: HEK-293T was inoculated into a 12-well cell culture plate and cultured overnight. Before protein treatment, it was ensured that the number of cells per well was about 2^{∗}10⁶; the cells were rinsed three times with serum-free DMEM medium, and then the delivery system protein TINNeL-eGFP and the control protein TINNe-eGFP respectively at 5 µM were added to serum-free medium; after incubation for 3 h, the pre-cooled serum-free DMEM containing 20 U/mL heparin was used to rinse for three times to remove the proteins that were adsorbed on the cell membrane but failed to enter the cells, and then the cells were collected for flow cytometry analysis. We found that the fluorescence intensity of TINNeL-eGFP after dimerization was 4 times higher, that was, the endocytosis efficiency was significantly improved after the addition of the leucine zipper domain (FIG. 9)..

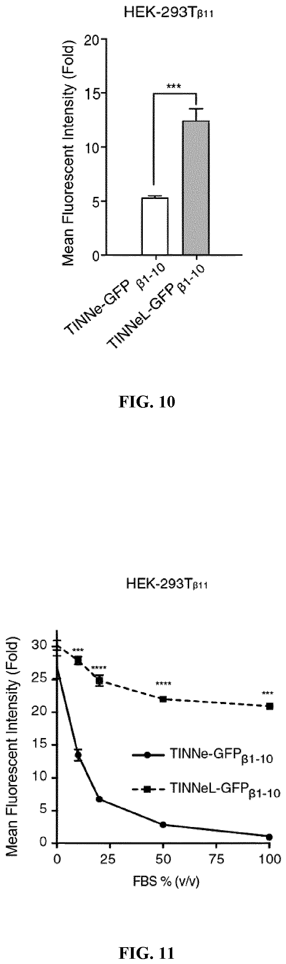

Evaluation of vesicle escape efficiency: We used the Split-GFP system to further evaluate the delivery efficiency of the TINNeL system by observing changes in its escape efficiency. HEK-293T β11 were inoculated into a 12-well cell culture plate and cultured overnight. Before protein treatment, it was ensured that the number of cells in each well was about 2^{∗}10⁶; the cells were rinsed three times with serum-free DMEM medium, and then the delivery system protein TINNeL-GFP β1-10 and the control protein TINNe-GFP β1-10 respectively at 1 µM were added to serum-free medium; after incubation for 3 h, the pre-cooled serum-free DMEM containing 20 U/mL heparin was used to rinse for three times to remove the proteins that were adsorbed on the cell membrane but failed to enter the cells. The culturing was continued for another 9 h after changing the medium to serum-containing medium (10 % FBS), and the cells were collected for flow cytometry analysis. The results were shown in FIG. 10, in which TINNeL-GFP β1-10 had higher fluorescence intensity, indicating that the TINNeL delivery system could improve both endocytosis efficiency and vesicle escape efficiency, and was a more efficient delivery method.

### 2.4 Evaluation of delivery efficiency of TINNeL delivery system under serum conditions

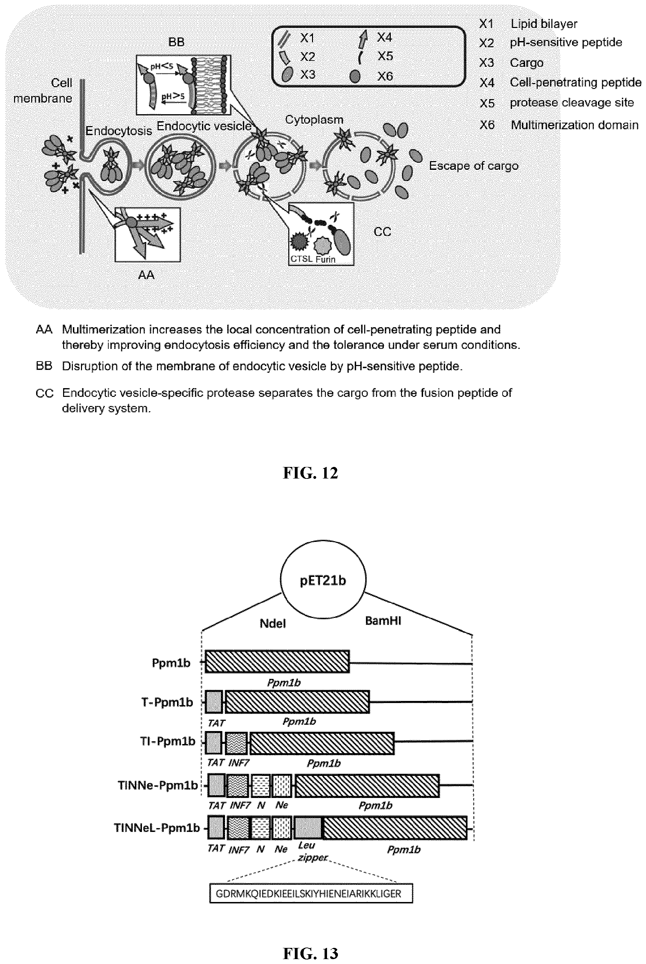

HEK-293T cells were inoculated into a 12-well cell culture plate and cultured overnight, before protein treatment, it was ensured that the number of cells in each well was about 2^{∗}10⁶; the cells were rinsed three times with serum-free DMEM medium, and TINNeL-GFP β1-10 and the control protein TINNe-GFP β1-10 respectively at 5 µM were added to serum-free, 10%, 20%, 50%, and 100% FBS DMEM media; after incubation for 3 h, the pre-cooled serum-free DMEM containing 20 U/mL heparin was used to rinse for four times to remove the proteins that were adsorbed on the cell membrane but failed to enter the cells. The culturing was continued for another 9 h after changing the medium to serum-free DMEM medium, then the cells were collected for flow cytometry analysis. The results were shown in FIG. 11. With the increase of serum content, the fluorescence intensity of TINNeL-GFP β1-10 and TINNe-GFP β1-10 groups decreased, but TINNeL-GFP β1-10 was less affected. Under the condition of 100% FBS concentration, the green fluorescence signal of TINNe-GFP β1-10 group could hardly be detected, while TINNeL-GFP β1 - 10 still had 70% efficiency compared with that without serum. TINNeL not only had high efficiency delivery ability, and its delivery efficiency would not be greatly affected in the presence of serum. FIG. 12 shows a schematic diagram of the principle of the TINNeL delivery system.

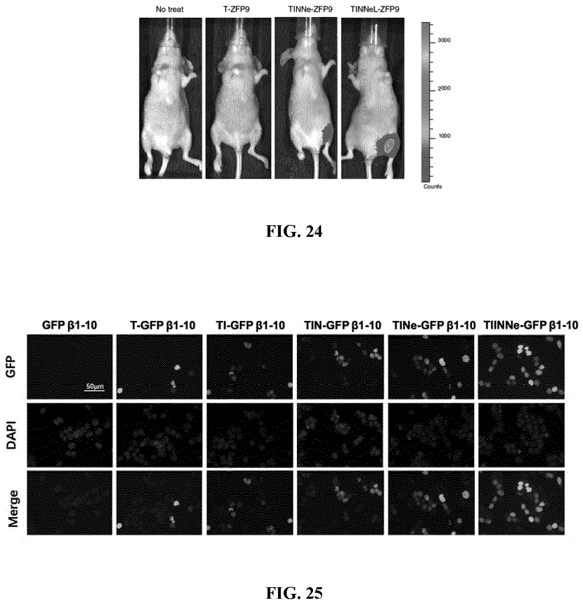

In addition, the present inventors also evaluated the vesicle escape efficiency of the delivery system containing protease recognition sequence N (TIN-GFP β1-10) and the delivery system containing protease recognition sequence Ne (TINe-GFP β1-10) based on the Split-GFP system. The delivery systems described above differed from TINNe-GFP β1-10 only in that the protease recognition sequence was replaced by N or Ne alone. The results were shown in FIG. 25. Compared with TI-GFP β1-10 or TIN-GFP β1-10 without protease recognition sequence, either the delivery system (TIN-GFP β1-10) that contained the protease recognition sequence N alone, or the delivery system (TINe-GFP β1-10) that contained the protease recognition sequence Ne, the escape efficiency of endocytic vesicles was significantly improved. The above results showed that either the protease recognition sequence N or Ne alone had the ability to improve the vesicle escape efficiency.

### Example 3: Application of TINNeL-Ppmlb in inhibiting apoptosis induced by TNF-α

Cell death can be divided into apoptosis and necrosis. Among them, cell necrosis may lead to cell membrane rupture, swelling and leakage of contents, resulting in a severe inflammatory response, which is mainly controlled by receptor-interaction kinase 3 (RIP3). Under the stimulation of TNF-α, necrosomes containing Rip1 and Rip3 are formed in cells, and Rip3 in the necrosomes recruits and phosphorylates Mlkl. Phosphorylated Mlk1 translocates to the cell membrane to perform necroptosis, during which the phosphorylation of Rip3 is necessary for the recruitment of Mlk1 to necroptosis. Therefore, the phosphorylation of Rip3 is likely an important target to inhibit cell necrosis and control the inflammatory response caused thereby. Studies have shown that protein phosphatase 1B (Ppm1b) can inhibit necroptosis in cultured cells and mice by dephosphorylating Rip3, and Ppm1b protein has become an important tool to control programmed necrosis. Given that programmed cell necrosis has been found to be closely related to the occurrence of inflammatory diseases, ischemia-reperfusion injury, neurodegenerative diseases and other diseases, Ppm1b protein has shown great potential in the treatment of the above-mentioned diseases related to programmed cell necrosis.

### 3.1 Construction of expression vectors for TINNeL-Ppm1b complex and other control protein

Expression vectors for recombinant protein containing TAT, INF7, protease cleavage site, multimerization domain, and cargo molecule Ppm1b (SEQ ID NO: 29) were constructed. The structural schematic diagram of each recombinant protein was shown in FIG. 13, the amino acid sequence of each component from the N-terminus to the C-terminus was shown in the table below. The construction method was as follows: the nucleic acid sequences of TAT, INF7, N, Ne, and Ppm1b were obtained by PCR amplification, and these parts were connected by multiple rounds of PCR, and in the last round of PCR, the *NdeI* restriction site and the overlap on upstream of the corresponding *NdeI* restriction site on pET-21b(+) were introduced at the 5' end of the fragment by forward primer, and the *BamHI* restriction site and the overlap on downstream of the corresponding *BamHI* restriction site on pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly.

**Table 5: Components of delivery system-cargo molecule complex**

| Name of complex | N-terminal → C-terminal components and sequences thereof | | | | |
|---|---|---|---|---|---|
| | CPP | pH-sensitive peptide | Protease recognition sequence | Leucine zipper | Cargo |
| Ppm1b | None | None | None | None | Ppm1b SEQ ID NO: 29 |
| T- Ppm1b | Tat SEQ ID NO:14 | | | | |
| TI- Ppm1b | | INF7 SEQ ID NO: 12 | | | |
| TINNe-Ppm1b | | | N+Ne | | |
| TINNeL-Ppm1b | | | | SEQ ID NO: 1 | |

### 3.2 Expression and purification of TINNeL-Ppmlb protein and other control proteins

First, the expression plasmid obtained in 3.1 was transformed into the expression strain *E.coli* BL21 (DE3); a single colony was picked from the transformed plate and inoculated into 5ml of LB liquid medium containing ampicillin resistance and cultured overnight, and 1ml of the bacterial liquid overnight cultured was taken and transferred to 500 ml of LB liquid medium containing ampicillin resistance, cultivated at 37°C and 180rpm until the bacterial liquid had an OD₆₀₀ of about 0.6, and then inducer IPTG was added to a final concentration of 0.2mM, and induction was performed at 25°C for 8 hours; after the induction expression was completed, the cells were collected by centrifugation under 7000 g at 4°C for 10 minutes, a part of the cells was taken to detect the induced expression of protein; then, the cells were resuspended with 10ml of protein purification equilibration buffer (50 ml C₃H₈O₃, 3.6342 g Tris(Hydroxymethyl)aminomethane was dissolved in 1 L double distilled water, and adjusted to pH 8.0), and disrupted by ultrasonication. Then, the supernatant was collected by centrifugation and loaded onto the Sulphopropyl (SP) cation exchange column of the AKTA protein purification system; then the equilibration buffer and high-salt eluent (50 ml C₃H₈O₃, 116.88 g NaCl, 3.6342 g Tris(Hydroxymethyl)aminomethane was dissolved in 1L of double-distilled water, and adjusted to pH 8.0) at different ratios were used to perform gradient elution to obtain the target protein. The protein concentration could be determined according to spectrophotometer or BCA protein concentration assay kit. Each purified fusion protein was aliquoted and stored at -20°C.

### 3.3 Effect of TINNeL-Ppmlb on TNF-α-induced necrosis of L292 cells

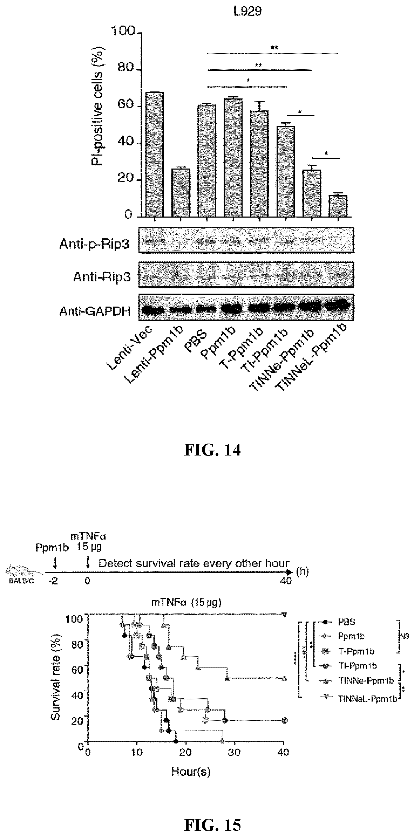

L929 cells were inoculated into a 12-well cell culture plate and cultured overnight. Before protein treatment, it was ensured that the number of cells in each well was about 2^{∗}10⁶, the cells were rinsed three times with serum-free DMEM medium, and then the delivery system protein TINNeL-Ppmlb and other control proteins were added respectively in serum-free medium. After 3 hours of incubation, 1 ml of 20 ng/ml TNF-α and 20 mM z-VAD diluted with 10% FBS DMEM were added. After 10 hours of incubation, the cells were collected for PI staining. The ratio of cell necrosis in cells was observed by flow cytometry analysis. At the same time, lentivirus-transduced Ppm1b (Lenti-Ppm1b) and lentivirus (Lenti-vec) were used as controls, the lentiviruses with Ppm1b expression sequences and the control lentiviruses were packaged and collected on HEK-293T cells, and after infecting L929 cells for 24 h to make Ppm1b fully expressed in the cells, the infected L929 cells were re-plated for later TNF-α stimulation. The results were shown in FIG. 14. In the L929 treated with TINNeL-Ppmlb, the ratio of cell necrosis caused by TNF-α was the lowest, that was only about 10%, and its inhibitory effect was even better than that of the lentivirus infection group (20%) as a positive control.

At the same time, because Ppm1b inhibited cell necrosis by inhibiting the process of Rip3 phosphorylation, we tried to verify the contents of Rip3 and phosphorylated Rip3 (p-Rip3) in L929 cells in different treatment groups (by the same treatment method as above) by western blotting, so as to further determine that TINNe-Ppmlb inhibited the necrosis of TNF-α cells by inhibiting the phosphorylation of Rip3 more efficiently after entering the cytoplasm. The results were shown in FIG. 14, in which the contents of Rip3 in L929 cells of all treatment groups were basically the same, while for p-Rip3 in the phosphorylated state, the TINNeL-Ppmlb group showed a result significantly lower than those of other protein groups, which indicated that TINNeL-Ppm1b could more efficiently enter L929 cytoplasm and inhibit the necrosis caused by TNF-α by inhibiting the phosphorylation of Rip3.

### 3.4 Evaluation of inhibitory activity of TINNeL-Ppmlb on TNF-α-induced systemic inflammatory response syndrome

In mice, TNF-α-induced cell necrosis further triggers a systemic inflammatory response syndrome (SIRS) in mice and ultimately leads to mouse death. The TINNeL delivery system not only had high delivery efficiency, but also should be able to maintain a high delivery efficiency in the presence of serum, that was, it could still exert its delivery efficiency well in vivo. Therefore, we attempted to observe whether TINNeL-Ppmlb still had an ideal delivery efficiency in vivo based on TNF-α-induced SIRS model.

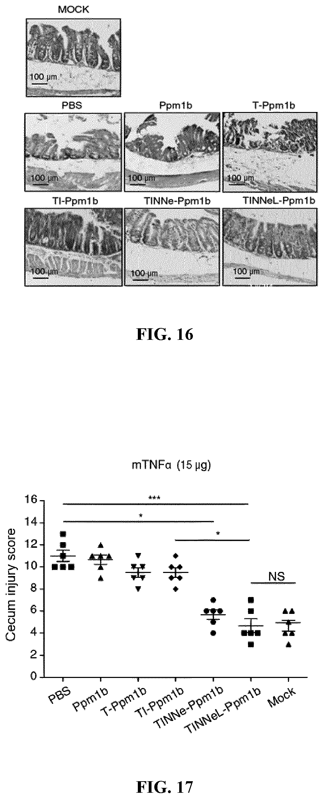

Through the tail vein route, 25 mmol of TINNeL-Ppmlb and other control proteins were injected into 6-week-old female BALB/C mice (12 per group), and 15 µg of TNF-α was also injected into the mice through the tail vein route 2 hours later, and the survival of the mice was observed every other hour thereafter (FIG. 15). The results showed that during the observation period (the survival rate was basically stable after 40 h), all the mice in the control group died of inflammation caused by TNF-α within 20 h, while the mice in the TINNeL-Ppmlb group were still 100% alive at the end of the observation period (FIG. 15). At the same time, TNF-α-induced SIRS could be directly observed from the degree of cecum injury in mice, so we injected 25 nmol of TINNeL-Ppmlb and other control proteins into 6-week-old female BALB/C mice (6 per group) through the tail vein route, 15 µg of TNF-α was also administered to the mice through the tail vein 2 hours later, and the mice were euthanized 10 hours later, and the cecum was removed for HE staining to observe the cecum damage of the mice in different treatment groups and the score of damage was given. The results showed that the mice in the TINNeL-Ppmlb group had almost uninjured cecum (FIG. 16), and the score was comparable to that of the Mock group (the control group that did not receive TNF-α), and could more effectively inhibit TNF-α-induced SIRS as compared with other proteins (FIG. 17). Therefore, based on the above results, we confirmed that TINNeL-Ppmlb could effectively inhibit TNF-α-induced SIRS in mouse, and TINNeL was a more effective delivery system.

### Example 4: Application of TINNeL-Ppm1b in treatment of acute liver injury caused by APAP

Acetaminophen (APAP) is a commonly used antipyretic and analgesic drug in clinical practice, but its overdose can lead to acute liver injury and the risk of death. In recent years, studies have confirmed that acute liver injury induced by APAP is caused by an inflammatory response induced by programmed cell necrosis mediated by Rip3. Therefore, dephosphorylation of Rip3 by Ppm1b is very likely to achieve the effect of treating APAP-induced acute liver injury.

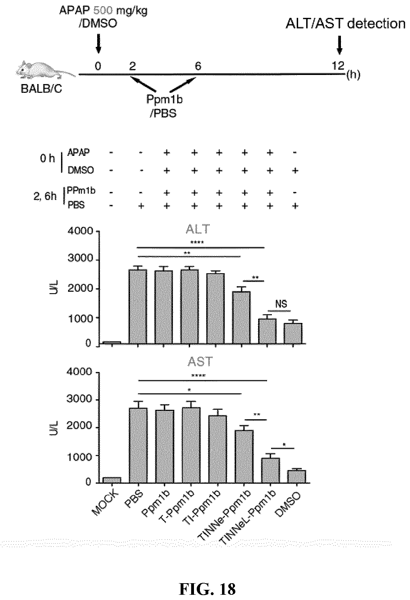

First, the mice aged 5 to 7 weeks (16-18 g) were injected with 500 mg/kg (non-lethal dose) of APAP through the tail vein route, and two injections of 25 nmol of TINNeL-Ppm1b and other control proteins prepared in Example 3 (7 mice in each group) were injected through the tail vein route 2 h and 6 h later, respectively; the blood sample was collected by orbital bleeding 6 h after APAP injection (12 h), the levels of ALT and AST in serum were detected, and the liver damage of the mice was evaluated. The results showed that the ALT and AST levels of the TINNeL-Ppmlb group were the lowest, which were basically the same as those of the APAP dilution buffer DMSO group (the increase of ALT/AST was caused by DMSO, and this part was not produced by the Rip3 pathway). Therefore, TINNeL- Ppm1b could achieve an ideal therapeutic effect of the APAP-induced acute liver injury (FIG. 18).

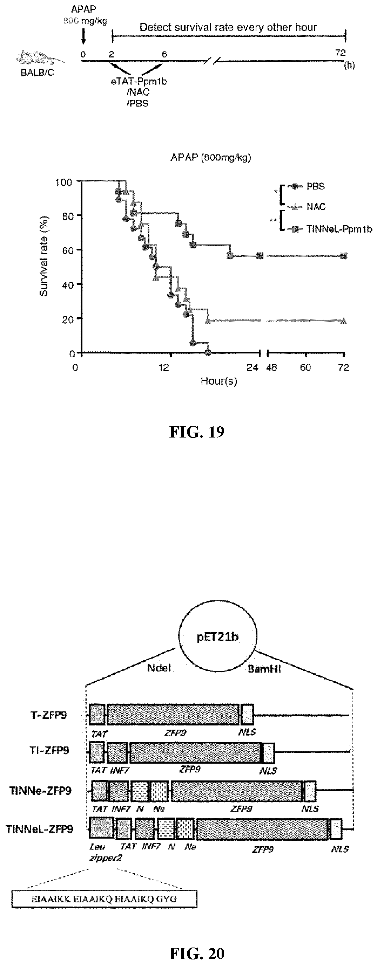

If the dose of APAP was further increased, it could lead to more severe liver damage and then induce the death of mice. Therefore, we tried to observe whether TINNeL-Ppmlb could treat the liver damage of mice at a lethal dose of APAP. Meanwhile, we used NAC, the only drug currently approved by FDA for the treatment of APAP-induced liver injury, as a control. First, BALB/C mice (16-18 g) aged 5 to 7 weeks were injected with 800 mg/kg (lethal dose) of APAP through the tail vein route, and then two injections of 25 mmol of TINNeL-Ppmlb, NAC, PBS (7 mice in each group) were injected through the tail vein route 2 h and 6 h later, respectively; and then the survival of mice was observed every other hour. The results were shown in FIG. 19. All the mice in the PBS group died within 18 h. During the observation period of 72h (acute phase occurred within 24 h), the survival rate of the mice in the NAC group was 20%, while the TINNeL-Ppmlb group could reach 60%. Therefore, even for more severe acute liver injury caused by APAP, TINNeL-Ppmlb showed a good therapeutic effect, which was even better than the current clinical drug NAC. The above results fully demonstrated the advantages of TINNeL in delivery in vivo.

### Example 5: Evaluation of nucleic acid delivery efficiency in vitro and in vivo of TINNeL-ZFP9

5.1 Construction of expression vectors for TINNeL-ZFP9 and other control protein The expression vectors of recombinant protein containing TAT, INF7, protease cleavage site, multimerization domain, cargo molecule ZFP9 (SEQ ID NO: 31) were constructed, the structure of each recombinant protein was shown in FIG. 20, and the amino acid sequence of each component from the N-terminus to the C-terminus was shown in the following table. The construction method was as follows: the nucleic acid sequences encoding TAT, INF7, protease cleavage site, leucine zipper, ZFP9 were obtained by PCR amplification, and these parts were ligated by multiple rounds of PCR, and in the last round of PCR process, the *NdeI* restriction site and the overlap on upstream of the corresponding *NdeI* restriction site on pET-21b(+) were introduced at the 5' end of the fragment through the forward primer, and the *BamHI* restriction site and the overlap on downstream of the corresponding *BamHI* restriction site on pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly.

**Table 6: Components of delivery system-cargo molecule complex**

| Name of complex | N-terminal → C-terminal components and sequences thereof | | | | |
|---|---|---|---|---|---|
| | Leucine zipper | CPP | pH-sensitive peptide | Protease recognition sequence | Cargo |
| T-ZFP9 | None | Tat SEQ ID NO:14 | None | None | ZFP9-NLS SEQ ID NO: 31 |
| TI-ZFP9 | | | INF7 SEQ ID NO: 12 | | |
| TINNe-ZFP9 | | | | N+Ne | |
| TINNeL-ZFP9 | SEQ ID NO: 2 | | | | |

### 5.2 Expression and purification of TINNeL-ZFP9 and control proteins

First, the expression plasmid obtained in 5.1 was transformed into the expression strain *E.coli* BL21 (DE3); a single colony was picked from the transformed plate and inoculated into 5ml of LB liquid medium containing ampicillin resistance and cultured overnight, and then 1ml of the bacterial liquid overnight cultured was taken and transferred to 500 ml of LB liquid medium containing ampicillin resistance, cultivated at 37°C and 180 rpm until the bacterial liquid had an OD₆₀₀ of about 0.6, and then inducer IPTG was added to a final concentration of 0.2mM, and induction was performed at 25°C for 8 hours; after the induction expression was completed, the cells were collected by centrifugation under 7000g at 4°C for 10 minutes, and a part of the cells was taken to detect the induced expression of protein; then, the cells were resuspended with 10ml of protein purification equilibration buffer (50 ml C₃H₈O₃, 3.6342 g Tris(Hydroxymethyl)aminomethane was dissolved in 1L double distilled water, and adjusted to pH 8.0), and disrupted by ultrasonication. Then the supernatant was collected by centrifugation and loaded onto the Sulphopropyl (SP) cation exchange column of the AKTA protein purification system; then the equilibration buffer and high-salt eluent (50 ml C₃H₈O₃, 116.88 g NaCl, 3.6342 g Tris(hydroxymethyl)aminomethane was dissolved in 1L of double-distilled water, and adjusted to pH 8.0) at different ratios were used to perform gradient elution to obtain the target protein. The protein concentration could be determined according to spectrophotometer or BCA protein concentration assay kit. Each purified fusion protein was aliquoted and stored at -20°C.

### 5.3 Construction of eukaryotic expression plasmid containing ZFP9 binding site

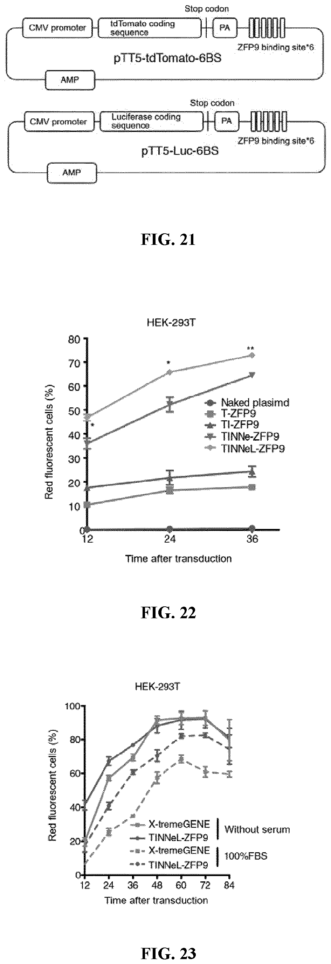

The expression vector (pTT5-tdTomato-6BS plasmid) containing tdTomato coding sequence and ZFP9 binding site was constructed, and its schematic structure was shown in FIG. 21. The tdTomato coding sequence (SEQ ID NO: 33) and the sequence of ZFP9 binding sites (6^{∗} binding sites) (SEQ ID NO: 34) were obtained by amplification through PCR, and the two were ligated by two rounds of PCR, and during the second round of PCR, the *Hind III* restriction site and the overlap on upstream of the corresponding *Hind III*restriction site on pTT5 vector were introduced at the 5' end of the fragment through the forward primer, and the BamHI restriction site and the overlap on downstream of the corresponding *BamHI* restriction site on pTT5 vector were introduced at the 3' end of the fragment through the reverse primer. The pTT5 plasmid was double-digested with *Hind III* and *BamH I.* The insert fragments with overlaps were ligated to the digested pTT5 vector by GIBSON assembly.

### 5.4 Evaluation of delivery efficiency of TINNeL-ZFP9 in delivery of plasmid DNA

First, HEK-293T cells were inoculated into a 12-well plate and cultured overnight. Before protein treatment, it was ensured that the number of cells in each well was about 5^{∗}10⁶; 5 µM of the delivery system protein or other control proteins and 5 g pTT5-tdTomato-6BS plasmid were co-incubated at 37°C for 30 min to fully form a complex; the cells were rinsed three times with serum-free DMEM medium, and then the complex was added and incubated for 3 h; the medium was replaced with 10% FBS DMEM and the culturing was continued, and the red fluorescent protein expression was observed by flow cytometry analysis after the medium was replaced for 12 h, 24 h, and 36 h respectively. The results were shown in FIG. 22. Compared with other control proteins, the red fluorescence ratio of the TINNeL-ZFP9 group was the highest at the three detection time points. Therefore, TINNeL-ZFP9 was more effective at delivering pTT5-tdTomato-6BS attached to it into the nucleus, thereby realizing the expression of the red fluorescent gene on the plasmid.

### 5.5 Evaluation of delivery efficiency of TINNeL-ZFP9 under serum conditions

In this example, the transfection effect of TINNeL-ZFP9 under serum conditions was investigated, and the commercial transfection reagent X-tremeGENE, which could work under serum conditions, was used as a control.

First, HEK-293T cells were inoculated into a 12-well plate and cultured overnight. Before protein treatment, it was ensured that the number of cells in each well was about 5^{∗}10⁶; 5 µM of TINNeL-ZFP9 and 5 µg pTT5-tdTomato-6BS plasmid was co-incubated at 37°C for 30 min to fully form a complex; the complex was added under 100% FBS serum conditions and serum-free conditions and incubated for 3 hours; the medium was replaced with 10% FBS DMEM and the culturing was continued; in the control X-tremeGENE group, 5 µg of pTT5-tdTomato-6BS plasmid and X-tremeGENE were mixed at a ratio of 1 µg DNA:3 µl X-tremeGENE, then allowed to stand at room temperature for 30 min, the incubation was performed under 100% FBS serum conditions and serum free conditions for 8 h, and then the medium was replaced with 10% FBS DMEM and the culturing was continued. For the above treatment groups, the expression of red fluorescent protein was observed by flow cytometry analysis after the medium was replaced for 12 h, 24 h, 36 h, 48 h, 60 h, 72 h, and 84 h, respectively. The results were shown in FIG. 23. Under serum-free conditions, the proportion of red fluorescent cells in the TINNeL-ZFP9 group increased faster, but after reaching the plateau phase, the proportion was basically the same as that in the X-tremeGENE group. However, under the condition of 100% FBS, there was a difference of nearly 10% in the proportion of red fluorescent cells at each time point, indicating that TINNeL-ZFP9 showed a more efficient transfection ability under serum conditions.

### 5.5 Evaluation of delivery efficiency of TINNeL-ZFP9 in mice

The pTT5-Luc-6BS expression plasmid with Luciferase encoding gene (SEQ ID NO:35) and ZFP9 binding site (SEQ ID NO:34) was constructed, and the construction method was the same as the above 5.3. After 4 nmol of TINNeL-ZFP9 or other control proteins TINNe-ZFP9, T-ZFP9 were mixed with 5 µg pTT5-Luc-6BS expression plasmid for 30 minutes, it was injected into the left leg muscle of nude mice, and the fluorescence intensity was imaged and analyzed 24 hours later. The results were shown in FIG. 24. The T-ZFP9 and no-treatment groups had almost no fluorescence detected, while the TINNe-ZFP9 had weak fluorescence, while the TINNeL-ZFP9 group had strong fluorescence detected. Therefore, it was confirmed in mouse that TINNeL- ZFP9 could efficiently bind and deliver DNA.

Although specific embodiments of the present invention have been described in detail, those skilled in the art will appreciate that various modifications and changes can be made to the details in light of all the teachings that have been published, and that these changes are all within the scope of the present invention. The full division of the present invention is given by the appended claims and any equivalents thereof.

## Claims

1. A fusion polypeptide, which comprises a multimerization domain sequence, a cell-penetrating peptide, a pH-sensitive fusogenic peptide, and a protease recognition sequence.

2. The fusion polypeptide according to claim 1, wherein the multimerization domain is a dimerization domain, a trimerization domain, a tetramerization domain, or any higher-order multimerization domain;
preferably, the multimerization domain is selected from the group consisting of: leucine zipper, NOE, GCN4-P1, Delta and any combination thereof;
preferably, the multimerization domain is selected from leucine zipper;
preferably, the multimerization domain sequence comprises the sequence shown in SEQ ID NO: 1 or 2.

3. The fusion polypeptide according to claim 1 or 2, wherein the cell-penetrating peptide is selected from the group consisting of Penetratin, a Tat-derived peptide (e.g., Tat(48-60) or Tat(47-57)), Rev (34-50), VP22, transportan, Pep-1, Pep-7, and any combination thereof;
preferably, the cell-penetrating peptide comprises a Tat-derived peptide, such as Tat(48-60); preferably, the cell-penetrating peptide comprises the sequence shown in SEQ ID NO: 14.

4. The fusion polypeptide according to any one of claims 1 to 3, wherein the pH-sensitive fusogenic peptide is selected from the group consisting of influenza virus HA2 or mutant thereof (e.g., INF7, KALA or GALA), melittin, and any combination thereof;
preferably, the pH-sensitive fusogenic peptide comprises INF7;
preferably, the pH-sensitive fusogenic peptide comprises the sequence shown in SEQ ID NO:12.

5. The fusion polypeptide according to any one of claims 1 to 4, wherein the protease is selected from furin and/or lysosomal cysteine protease;
preferably, the protease recognition sequence is selected from the group consisting of furin recognition sequence, lysosomal cysteine protease recognition sequence, and combination thereof.

6. The fusion polypeptide according to claim 5, wherein the furin recognition sequence comprises R-X₁-X₂-R (SEQ ID NO: 47), wherein X₁ is any amino acid, and X₂ is K or R;
preferably, the furin recognition sequence comprises R-R-X₁-X₂-R (SEQ ID NO: 48);
preferably, the furin recognition sequence comprises the sequence shown in SEQ ID NO: 49;
preferably, the furin recognition sequence comprises the sequence shown in SEQ ID NO: 8.

7. The fusion polypeptide according to claim 5 or 6, wherein the lysosomal cysteine protease is selected from the group consisting of cathepsin B, cathepsin C, cathepsin X, cathepsin S, cathepsin L, cathepsin D or cathepsin H;
preferably, the lysosomal cysteine protease is cathepsin L;
preferably, the cathepsin L recognition sequence comprises the sequence shown in SEQ ID NO: 10.

8. The fusion polypeptide according to any one of claims 1 to 7, wherein the protease recognition sequence comprises a furin recognition sequence and a cathepsin L recognition sequence;
preferably, the protease recognition sequence comprises SEQ ID NO: 49 and SEQ ID NO: 10;
preferably, the protease recognition sequence comprises SEQ ID NO: 8 and SEQ ID NO: 10.

9. The fusion polypeptide according to any one of claims 1 to 8, wherein the fusion polypeptide comprises: the cell-penetrating peptide, the pH-sensitive fusogenic peptide, the protease recognition sequence from the N-terminus to the C-terminus, or, comprises: the pH-sensitive fusogenic peptide, the cell-penetrating peptide, the protease recognition sequence from the N-terminus to the C-terminus; and the multimerization domain sequence is located at the N-terminus or the C-terminus of the fusion polypeptide, or between any two adjacent domains as above mentioned;
preferably, the protease recognition sequence comprises the furin recognition sequence and the cathepsin L recognition sequence from the N-terminus to the C-terminus, or comprises the cathepsin L recognition sequence and the furin recognition sequence from the N-terminus to the C-terminus.

10. The fusion polypeptide according to any one of claims 1 to 9, wherein any two adjacent domains contained in the fusion polypeptide are optionally linked by a peptide linker;
preferably, the peptide linker is (GₘS)ₙ, wherein m is selected from an integer of 1 to 4 and n is selected from an integer of 1 to 3.

11. The fusion polypeptide according to any one of claims 1 to 10, wherein the fusion polypeptide comprises the sequence shown in any one of SEQ ID NOs: 16 to 18.

12. A multimer of the fusion polypeptide according to any one of claims 1 to 11;
preferably, the multimer is a dimer, trimer or tetramer;
preferably, the multimer is a homomultimer.

13. A fusion protein, which comprises the fusion polypeptide according to any one of claims 1 to 11, and an additional polypeptide;
preferably, the additional polypeptide comprises a detectable label;
preferably, the additional polypeptide comprises an epitope tag, a protein sequence encoded by reporter gene and/or a nuclear localization signal (NLS) sequence.

14. The fusion protein according to claim 13, wherein the additional polypeptide is a nucleic acid binding domain sequence;
preferably, the nucleic acid binding domain sequence is a zinc finger protein (e.g.,ZFP9);
preferably, the nucleic acid binding domain sequence comprises the sequence shown in SEQ ID NO: 31;
preferably, the fusion protein comprises the sequence shown in any one of SEQ ID NOs: 19 to 21.

15. The fusion protein according to claim 13 or 14, wherein,
(i) the fusion protein comprises: the cell-penetrating peptide, the pH-sensitive fusogenic peptide, the protease recognition sequence and the additional polypeptide from the N-terminus to the C-terminus; and, the multimerization domain sequence is located at the N-terminus or the C-terminus of the fusion protein, or between any two adjacent domains described above; preferably, the protease recognition sequence comprises the furin recognition sequence and the cathepsin L recognition sequence from the N-terminus to the C-terminus, or comprises the cathepsin L recognition sequence and the furin recognition sequence from the N-terminus to the C-terminus; or,
(ii) the fusion protein comprises: the pH-sensitive fusogenic peptide, the cell-penetrating peptide, the protease recognition sequence, and the additional polypeptide from the N-terminus to the C-terminus; and, the multimerization domain sequence is located at the N-terminus or the C-terminus of the fusion protein, or between any two adjacent domains described above; preferably, the protease recognition sequence comprises the furin recognition sequence and the cathepsin L recognition sequence from the N-terminus to the C-terminus, or comprises the cathepsin L recognition sequence and the furin recognition sequence from the N-terminus to the C-terminus; or,
(iii) the additional polypeptide is fused to the C-terminus of the fusion polypeptide.

16. A multimer of the fusion protein according to any one of claims 13 to 15;
preferably, the multimer is a dimer, trimer or tetramer;
preferably, the multimer is a homomultimer.

17. An isolated nucleic acid molecule, which comprises a nucleotide sequence encoding the fusion polypeptide according to any one of claims 1 to 11, or the fusion protein according to any one of claims 13 to 15.

18. A vector, which comprises the isolated nucleic acid molecule according to claim 17.

19. A host cell, which comprises the isolated nucleic acid molecule according to claim 17 or the vector according to claim 18.

20. A method for preparing the fusion polypeptide according to any one of claims 1 to 11, or the fusion protein according to any one of claims 13 to 15, which comprises, culturing the host cell according to claim 19 under suitable conditions, and recovering the fusion polypeptide or the fusion protein from a cell culture, wherein the fusion polypeptide or the fusion protein exists as a multimer.

21. A complex, which comprises the multimer according to claim 12 or the multimer according to claim 16, and a cargo molecule;
preferably, the cargo molecule is selected from the group consisting of protein, nucleic acid, carbohydrate, lipid, chemical compound and any mixture thereof;
preferably, the cargo molecule is fused, chemically coupled or non-covalently linked to the multimer.

22. The complex according to claim 21, wherein the cargo molecule is a peptide or a protein; preferably, the cargo molecule is fused to the multimer.

23. The complex according to claim 21, wherein the cargo molecule is a nucleic acid;
preferably, the nucleic acid is selected from the group consisting of DNA molecule, RNA molecule, siRNA, antisense oligonucleotide, ribozyme, aptamer and any combination thereof;
preferably, the multimer is a multimer of the fusion protein according to claim 14.

24. The complex according to claim 21, wherein the multimer is chemically coupled to the cargo molecule;
preferably, the chemical coupling is achieved through a disulfide bond, a phosphodiester bond, a phosphorothioate bond, an amide bond, an amine bond, a thioether bond, an ether bond, an ester bond or a carbon-carbon bond.

25. The complex according to claim 21, wherein the multimer is non-covalently linked to the cargo molecule;
preferably, the multimer is electrostatically conjugated to the cargo molecule.

26. A pharmaceutical composition, which comprises the complex according to any one of claims 21 to 25, and a pharmaceutically acceptable carrier and/or excipient;
preferably, the cargo molecule is a pharmaceutically active agent or a detectable label.

27. Use of the complex according to any one of claims 21 to 25 or the pharmaceutical composition according to claim 26 in the manufacture of a medicament for the treatment of a disease; wherein the cargo molecule contained in the complex is capable of treating the disease;
preferably, the disease is a disease associated with programmed necrosis, and the cargo molecule comprises protein phosphatase 1B; preferably, the disease associated with programmed necrosis comprises liver injury (e.g., drug-induced liver injury), inflammatory disease, ischemia-reperfusion injury and/or neurodegenerative disease.

28. A method for treating a disease, comprising administering the complex according to any one of claims 21 to 25 to a subject in need thereof; wherein, the cargo molecule contained in the complex is capable of treating the disease;
preferably, the disease is a disease associated with programmed necrosis, and the cargo molecule comprises protein phosphatase 1B; preferably, the disease associated with programmed necrosis comprises liver injury (e.g., drug-induced liver injury), inflammatory disease, ischemia-reperfusion injury and/or neurodegenerative disease.

29. A kit, which comprises the fusion polypeptide according to any one of claims 1 to 11, the multimer according to claim 12, the fusion protein according to any one of claims 13 to 15, the multimer according to claim 16, the isolated nucleic acid molecule according to claim 17, the vector according to claim 18, the host cell according to claim 19, or the complex according to any one of claims 21 to 25;
preferably, the kit further comprises an instruction for transfection and/or intracellular delivery.

30. Use of the fusion polypeptide according to any one of claims 1 to 11, the multimer according to claim 12, the fusion protein according to any one of claims 13 to 15, the multimer according to claim 16, the isolated nucleic acid molecule according to claim 17, the vector according to claim 18, the host cell according to claim 19, or the complex according to any one of claims 21 to 25 as a delivery reagent.

31. A method for delivering a cargo molecule into a cell, comprising: contacting the cell with the complex according to any one of claims 21 to 25, wherein the cargo molecule is the cargo molecule contained in the complex;
preferably, the contacting of the cell with the complex is performed in vitro;
preferably, the cargo molecule is selected from the group consisting of nucleic acid, peptide or protein, carbohydrate, lipid, chemical compound and any mixture thereof; preferably, the nucleic acid is selected from the group consisting of DNA molecule, RNA molecule, siRNA, antisense oligonucleotide, ribozyme, aptamer, and any combination thereof.
